# EUROPEAN PATENT APPLICATION

(11) **EP 2 042 912 A1**
(43) Date of publication of application: **01.04.2009**
(21) Application number: 07790814.3
(22) Date of filing: 13.07.2007
(51) Int. Cl.: G02C 7/04

(54) **LIQUID AGENT FOR CONTACT LENS AND METHOD FOR HYDROPHILIZING CONTACT LENS BY USING THE SAME**

(30) Priority: 14.07.2006 JP 2006220589; 25.01.2007 JP 2007042914
(71) Applicant: Tomey Co., Ltd., Nagoya-shi, Aichi 451-0051 (JP); Celagix Res. Ltd., Yokohama-shi, Kanagawa 226-0026 (JP)
(72) Inventor: MATSUMOTO, Satoru, Nagoya-shi Aichi 451-0051 (JP); KATO, Yasuyuki, Nagoya-shi Aichi 451-0051 (JP); ANAN, Naoki, Nagoya-shi Aichi 451-0051 (JP); GOTO, Mitsuaki c/o Celagix Res. Ltd., Midori-ku, Yokohama-shi Kanagawa 226-0026 (JP); IWAMA, Masamichi c/o Celagix Res. Ltd., Midori-ku, Yokohama-shi Kanagawa 226-0026 (JP)
(74) Representative: Paget, Hugh Charles Edward
(86) International application number: PCT/JP2007/064034
(87) International publication number: WO 2008/007790

(57) **Abstract**

It is an object of the present invention to provide a contact lens solution that imparts stable hydrophilicity even to a surface of a contact lens having strong water repellency. There was prepared a contact lens solution comprising a copolymer of N-p-vinylbenzyl-D-lactonamide and at least one monomer copolymerizable therewith. The copolymer is present in the solution in an amount of 0.0001 to 5 w/v%.

## Description

### TECHNICAL FIELD

The present invention relates to a contact lens solution, particularly to a contact lens solution which can be advantageously used as a hydrophilizing agent used in performing hydrophilizing treatment to a surface of an oxygen-permeable hard or soft contact lens, a preservative solution used in maintaining hydrophilicity of a surface of a contact lens, or the like, and to a method for hydrophilizing a contact lens by using such a solution.

### BACKGROUND ART

The number of users of contact lenses is increasing year by year, and in association with this, research and development have been actively carried out for materials of contact lenses and lens design. Specifically, the contact lenses are used in direct contact with the cornea, unlike glasses used for the same purpose of vision correction, so that the contact lenses are required not to inhibit metabolism of the cornea while being worn. For this reason, development of contact lens materials excellent in oxygen permeability and research on lens design in which tear liquid exchange between the contact lenses and the cornea is smoothly carried out have been actively advanced.

For example, as polymers excellent in oxygen permeability, there are known silicone-based or fluorine-based polymers which are obtained by copolymerizing a siloxane compound or a fluorine-containing compound such as a siloxanyl (meth)acrylate, a polydimethylsiloxane macromer or a fluoroalkyl(meth)acrylate and one or two or more kinds of other monomers. These polymers have hitherto been widely used as contact lens materials.

However, such silicone-based or fluorine-based polymers generally have strong water repellency. Accordingly, in contact lenses made of such a silicone-based polymer or the like, the tear liquid is repelled on lens surfaces thereof, which has posed a problem that wearers have strong foreign-body sensation. Further, components in the tear liquid are liable to adhere to the contact lenses made of the silicone-based or fluorine-based polymer, so that a problem has been pointed out that adhesion of those components clouds the lenses and causes poor visibility of the wearers and the like.

In order to solve such problems caused by water repellency of the contact lenses, it is proposed that a hydrophilic monomer is used as the monomer to be copolymerized with the above-mentioned siloxane compound or the like, thereby imparting hydrophilicity to a finally obtained contact lens. However, the hydrophilic monomer is generally poor in compatibility with the above-mentioned silicon-containing compound or the like, so that when it is copolymerized, there is a risk of obtaining a clouded polymer. Further, when the amount of the hydrophilic monomer is increased in order to more improve hydrophilicity, there has been a problem of a decrease in stability of the lens standard and the like in the finally obtained contact lens.

In addition, methods for imparting hydrophilicity to a contact lens by performing various treatment to the contact lens are variously proposed. For example, there is proposed a method for imparting hydrophilicity to a contact lens by performing plasma treatment or plasma polymerization treatment to a surface of the contact lens (see patent documents 1 and 2). However, in the method for hydrophilizing the contact lens by such surface treatment, a specific treatment apparatus is necessary to perform it, and further, each of a front face and a back face of the contact lens has to be treated. This method has therefore had a problem with respect to productivity.

Further, methods for imparting hydrophilicity to the contact lens by immersing a contact lens in a solution containing specified compositions (hereinafter referred to as immersion methods) are widely known, and various solutions used therefor are proposed.

Specifically, there are proposed a method for immersing a contact lens in a solution containing three kinds of polymer compounds: polyvinyl alcohol, hydroxyethyl cellulose and polyvinylpyrrolidone as main components, a method for immersing a contact lens in a solution containing an ionically charged polymer material (patent document 3), a method for immersing a contact lens in a solution containing hydrolyzed collagen peptide (patent document 4), and the like. These methods are simple and easy, compared to the method for hydrophilizing the contact lens by surface treatment as described above. However, the degree of hydrophilicity which can be imparted to the contact lens is low, so that there has been a problem that it is difficult to impart desired hydrophilicity to the contact lens.

In addition to the above-mentioned solutions, various saccharide-containing solutions are proposed. For example, patent document 5 proposes a contact lens cleaning solution containing fine spherical particles of a polysaccharide, and patent document 6 proposes an ophthalmic solution containing a polysaccharide selected from the group consisting of dextrin and arabinogalactan. Further, patent document 7 discloses a contact lens preservative solution containing at least one selected from the group consisting of a monosaccharide and disaccharide, an enzyme and the like. Furthermore, patent document 8 discloses a contact lens solution containing alginic acid which is a straight-chain polysaccharide. All the saccharides used in these solutions are not modified, so that there is no problem in safety. However, as with the above-mentioned immersion methods using the other solutions, the degree of hydrophilicity which can be imparted to the contact lens is low, and hydrophilicity imparted to the contact lens is not maintained for a long period of time. Accordingly, a room for improvement has been left.

By the way, as a modified saccharide, there is known poly(N-p-vinylbenzyl-D-lactonamide) [hereinafter referred to as PVLA] which is a polystyrene derivative having a saccharide as side chains (see non-patent document 1). For such PVLA, there are proposed its use as a substrate in cell culturing (see patent documents 9 to 12), its use as a coating agent for a cell culture vessel (see patent document 13), its use in an antithrombogenic material (see patent document 14), its use as a DDS preparation in which a drug is enclosed with PVLA (see patent document 15), its use in a cosmetic (see patent document 16) and the like. All of the above use PVLA as a homopolymer.

Further, as an example of use of a copolymer of PVLA as a saccharide chain-containing styrene derivative and another monomer, there is proposed a method for incorporating a drug into a specified cell by using a graft copolymer of PVLA and a lysine oligomer having a methacryloyl group at an N-terminal (see patent document 17), a use of a copolymer of PVLA and 4-vinylbenzylhexadecanamide as an antithrombotic material (see patent document 18) and the like. Such an example of use of the saccharide side chain-containing polymer is proposed based on the fact that the saccharide side chain has a specific effect in cell culturing, or that the cell recognizes the saccharide side chain. However, as far as the present inventors know, there has been no example yet of such a saccharide side chain-containing polymer applied to the contact lens field.

Patent Document 1: JP-B-55-49288
Patent Document 2: JP-A-58-216222
Patent Document 3: JP-B-2-61017
Patent Document 4: JP-A-7-218878
Patent Document 5: JP-A-1-293314
Patent Document 6: JP-B-61-5452
Patent Document 7: JP-A-4-161921
Patent Document 8: JP-A-7-157747
Patent Document 9: JP-A-63-116692
Patent Document 10: JP-A-63-279787
Patent Document 11: JP-A-63-301786
Patent Document 12: JP-A-1-309681
Patent Document 13: JP-A-4-169530
Patent Document 14: JP-A-2-224664
Patent Document 15: JP-A-5-105637
Patent Document 16: JP-A-2000-273019
Patent Document 17: JP-A-10-45630
Patent Document 18: JP-A-2005-112987
Non-Patent Document 1: Kazukiyo Kobayashi et al., "Synthesis and Functions of Polystyrene Derivatives Having Pendant Oligosaccharides", Polymer Journal, Vol. 17, No. 4, pages 567 to 575 (1985)

### DISCLOSURE OF THE INVENTION

### PROBLEMS TO BE SOLVED BY THE INVENTION

The present invention has been made in the light of the situations discussed above. It is therefore, an object of the present invention to provide a contact lens solution which is capable of imparting stable hydrophilicity even to a surface of a contact lens having strong water repellency. It is another object of the present invention to provide a method for hydrophilzing a contact lens by which hydrophilizing treatment of a surface of a contact lens can be advantageously performed using such a contact lens solution.

### MEANS FOR SOLVING THE PROBLEMS

The object of the present invention is to provide a contact lens solution comprising a copolymer of N-p-vinylbenzyl-D-lactonamide and at least one monomer copolymerizable therewith, the copolymer being present in the solution in an amount of 0.0001 to 5 w/v%.

According to one preferred embodiment of the contact lens solution of the present invention, the above-mentioned copolymer is one selected from copolymers represented by the following formulas (1) to (4). 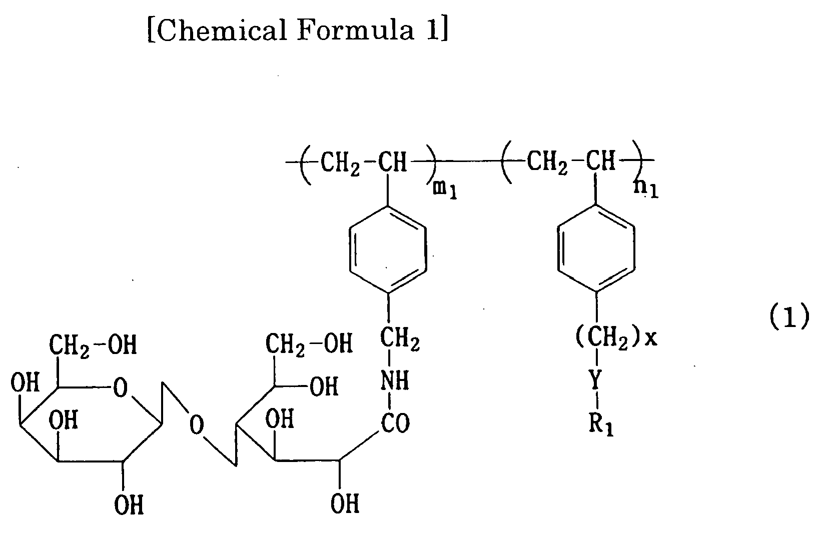 [wherein m₁:n₁=9:1 to 1:9; x represents an integer of 0 to 3; -Y- represents a single bond or an atomic group represented by -NHCO-, -NHCONH-, -CONH-, -OCONH-, -NHCOO-, -OCO-, -COO-, -CO- or -NH-; and R₁ is any one of the following (a) and (b) :
(a) a saturated or unsaturated i) monocycle, ii) bicycle or iii) five-membered or six-membered heterocycle containing O atom, S atom or N atom, which has 5 to 10 carbon atoms, wherein the monocycle, bicycle, five-membered or six-membered heterocycle may be at least partly substituted by at least one selected from the group consisting of a halogen atom, a hydroxy group, an amino group, a thiol group, a cyano group, a cyclopentyl group, a cyclohexyl group, a phenyl group and an acetyl group; and
(b) an alkyl group having 1 to 20 carbon atoms or an atomic group represented by general formula: -O-C_{α}H_{2α+1}, -S-C_{α}H_{2α+1} or -NH-C_{α}H_{2α+1} (in which α represents an integer of 1 to 20), wherein the alkyl group or atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group.] 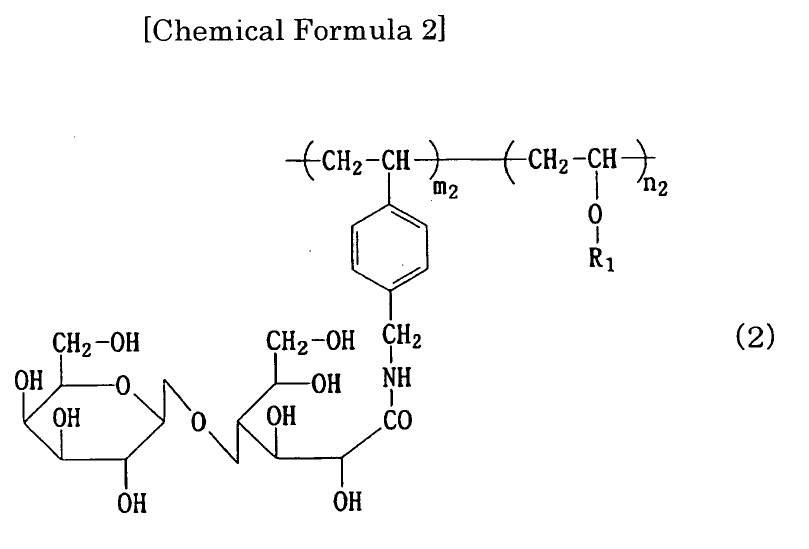
[wherein m₂:n₂=9:1 to 1:9, and R₁ has the same meaning as in formula (1).]

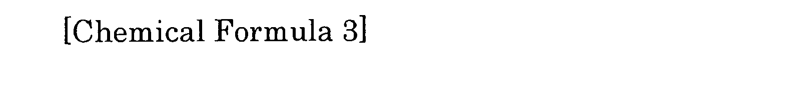

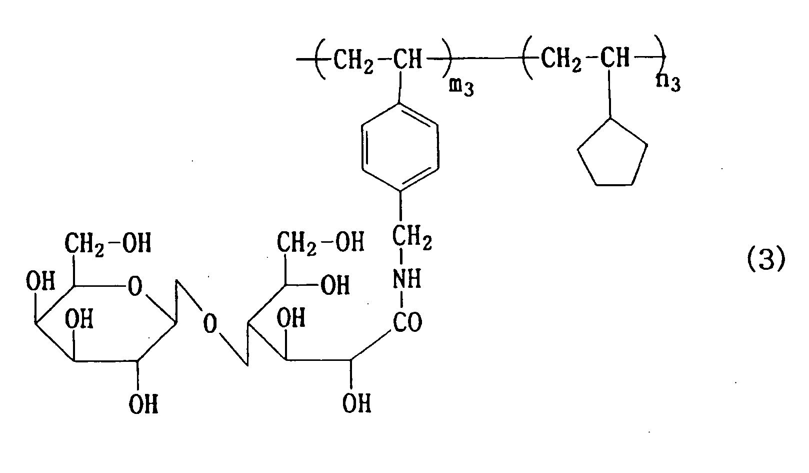

[wherein m₃:n₃=9:1 to 1:9.] 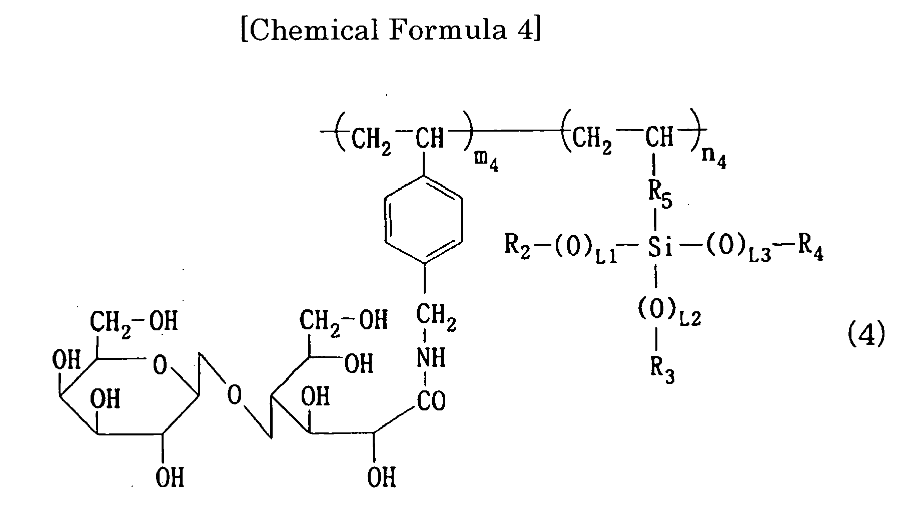 [wherein m₄:n₄=9:1 to 1:9; L1, L2 and L3 are each independently 0 or 1; and R₂, R₃ and R₄, which are independent from one another, are each an alkyl group having 1 to 20 carbon atoms or an atomic group represented by general formula: -O-C_{β}H_{2β+1}, -S-C_{β}H_{2β+1} or -NH-C_{β}H_{2β+1} (in which β represents an integer of 1 to 20), wherein the alkyl group or atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group, and -R₅- is any one of the following (a) and (b) :
(a) an atomic group represented by a single bond, or an atomic group represented by -NHCO-, -NHCONH-, -CONH-, -OCONH-, -NHCOO-, -OCO-, -COO-, -CO-, -NH- or -O-; and
(b) an atomic group represented by general formula: -C_{γ}H_{2γ}-, -O-C_{γ}H_{2γ}-, -C_{γ}H_{2γ}-O-, -S-C_{γ}H_{2γ}-, -C_{γ}H_{2γ}-S-, -NH-C_{γ}H_{2γ}- or -C_{γ}H_{2γ}-NH- (in which γ represents an integer of 1 to 20), wherein the atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group.]

According to another preferred embodiment of the contact lens solution of the present invention, the contact lens solution further comprises a nonionic surfactant in an amount of 0.001 to 10 w/v%.

According to still another preferred embodiment of the contact lens solution of the present invention, the contact lens solution further comprises a surfactant selected from the group consisting of an anionic surfactant, a cationic surfactant and an amphoteric surfactant, in an amount of 0.001 to 10 w/v%.

According to one desirable embodiment of the contact lens solution of the present invention, the contact lens solution further comprises at least one selected from the group consisting of a buffer, a tonicity agent, a thickener and a disinfectant.

In addition, it is another object of the present invention to provide a method for hydrophilizing a contact lens, comprising the step of bringing the contact lens solution according to any one of the above embodiments into contact with the contact lens.

In such a method for hydrophilizing a contact lens, advantageously, the contact lens is immersed in the contact lens solution, and then, the contact lens is rinsed.

### ADVANTAGEOUS EFFECT OF THE INVENTION

As described above, the contact lens solution according to the present invention contains the copolymer of N-p-vinylbenzyl-D-lactonamide and another monomer at the specified ratio, and such a copolymer has a hydrophilic moiety and a hydrophobic moiety at once. Accordingly, when the contact lens solution of the present invention is brought into contact with the contact lens, the copolymer contained in the solution is also well adsorbed into a surface of the contact lens having strong water repellency. A hydrophilic surface layer is formed on the surface of the contact lens by such adsorption, so that the contact lens is effectively hydrophilized.

Further, the copolymer contained in the solution is adsorbed into the surface of the contact lens by a hydrophobic bond which is a relatively strong bond, so that the contact lens to which hydrophilization treatment has been performed by using the contact lens solution of the present invention can enjoy its effect over a relatively long period of time.

Furthermore, the copolymer in the solution is adsorbed into the contact lens to form the surface layer on the contact lens, so that the occurrence of clouding on the surface of the contact lens, which sometimes occurs particularly at the time of use of the hard type contact lens, can be effectively prevented.

When the contact lens are hydrophilized according to the present invention by using the contact lens solution having such excellent characteristics as a hydrophilizing treatment agent, the contact lens after treatment exhibits excellent hydrophilicity over a long period of time.

### BRIEF DESCRIPTION OF THE DRAWINGS

FIG. 1 is a bar graph showing the averages of evaluations (average evaluation scores) of test subjects, respectively, which are obtained in "contact lens wear test I" of Examples.
FIG. 2 is a bar graph showing the averages of evaluations (average evaluation scores) of test subjects, respectively, which are obtained in "contact lens water wettability test II" of Examples.
FIG. 3 is a bar graph showing the averages of evaluations (average evaluation scores) of test subjects, respectively, which are obtained in "contact lens wear test II" of Examples when a commercially available contact lens is used as a test contact lens.
FIG. 4 is a bar graph showing the averages of evaluations (average evaluation scores) of test subjects, respectively, which are obtained in "contact lens wear test II" of Examples when another commercially available contact lens is used as a test contact lens.
FIG. 5 is a bar graph showing the averages of evaluations (average evaluation scores) of test subjects, respectively, which are obtained in "contact lens wear test II" of Examples when still another commercially available contact lens is used as a test contact lens.

### BEST MODE FOR CARRYING OUT THE INVENTION

The contact lens solution according to the present invention contains the copolymer comprising N-p-vinylbenzyl-D-lactonamide (hereinafter appropriately referred to as VLA) and at least one monomer copolymerizable therewith as essential components. In the synthesization of such a copolymer, VLA is first provided. As for synthesis methods of the VLA, one example thereof is described in non-patent document 1. The synthesis method of VLA disclosed in non-patent document 1 is as follows.

Initially, an equimolar mixture of p-vinylbenzyl chloride and potassium phthalimide is provided, and dissolved in N,N-dimethylformamide to form a reaction solution. This reaction solution is heated to allow p-vinylbenzyl chloride and potassium phthalimide to react with each other. The reaction solution after heating is concentrated under reduced pressure, and the residue containing a reaction product is dissolved in chloroform to form a chloroform solution. Such a chloroform solution is washed with an aqueous solution of sodium hydroxide and concentrated. Then, N-p-vinylbenzylphthalimide which is the reaction product is recrystallized by using methanol. N-p-vinylbenzylphthalimide thus obtained is dissolved in ethanol, and an ethanol solution of anhydrous hydrazine is added thereto, followed by reflux (to reflex). The resulting precipitate is separated by filtration, treated with an aqueous solution of sodium hydroxide and extracted with ether. The ether solution as an extract is dried, and then, concentrated under reduced pressure to obtain p-vinylbenzylamine. The flow of reaction up to here is shown below by a reaction formula.

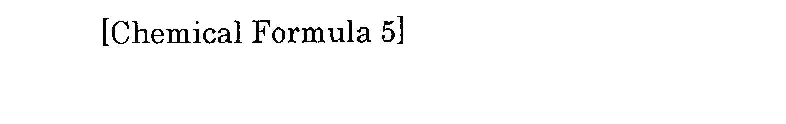

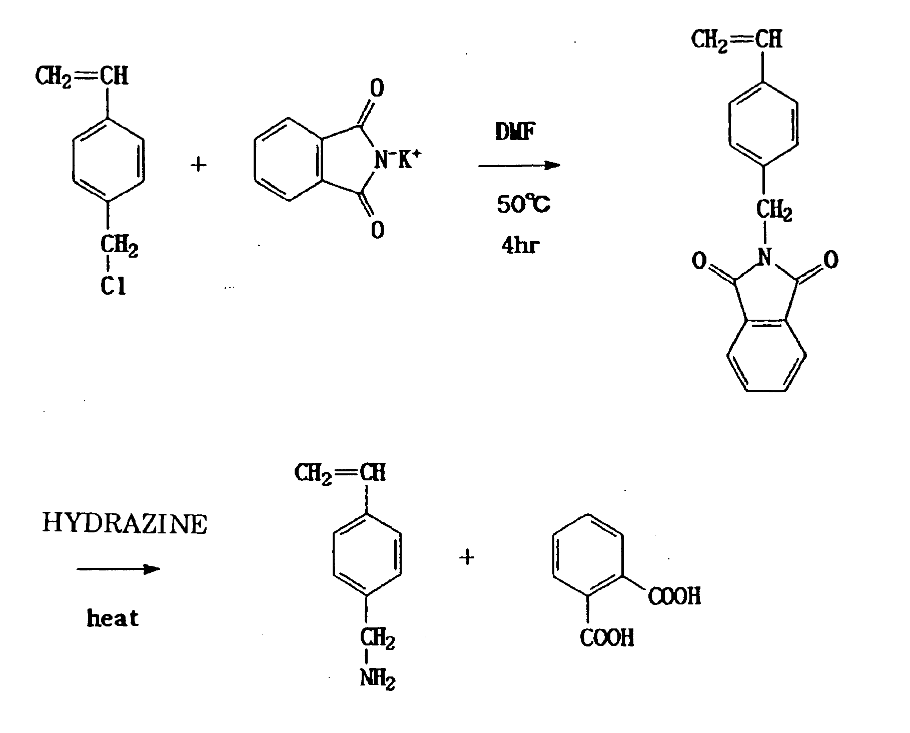

Meanwhile, O.B.D.galactopyranosyl-(1→4)-D-glucono-1,5-lactone, which is provided separately, is dissolved in methanol to form a methanol solution. A methanol solution of p-vinylbenzylamine described above is added to this methanol solution under heating. A white crystalline precipitate produced thereby is separated by filtration, and the residue is washed with cold methanol and dried under reduced pressure, whereby desired N-p-vinylbenzyl-D-lactonamide (VLA) is obtained. A chemical reaction formula of VLA formation is shown below as reference.

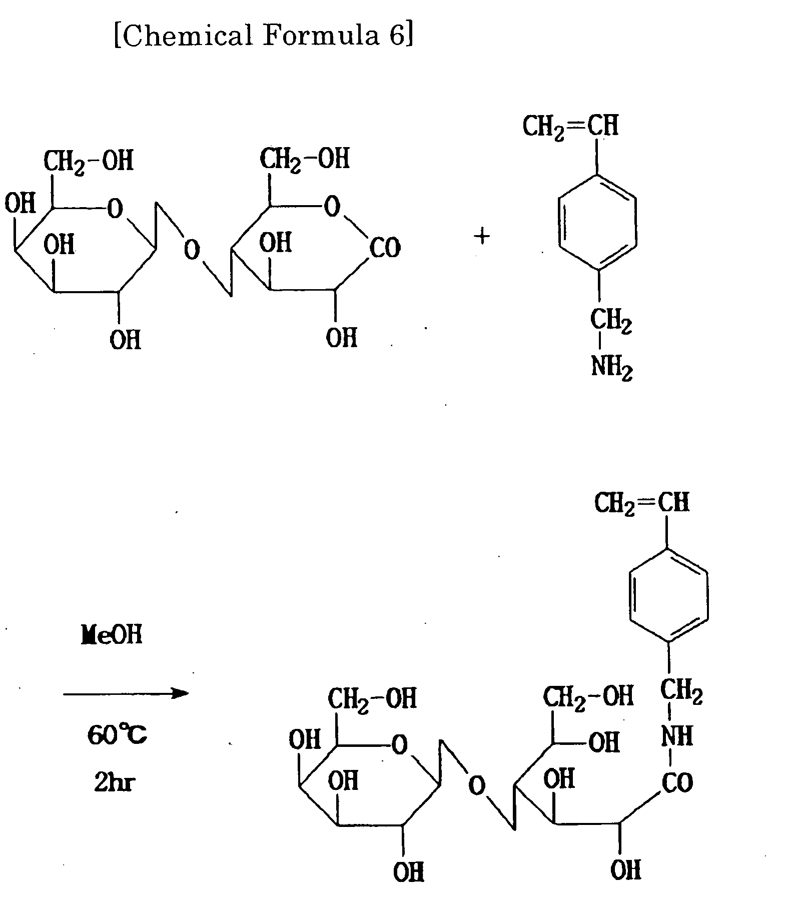

The synthesis method of VAL described in non-patent document 1 has been described above. However, in the present invention, it is to be understood that any VAL synthesized according to methods other than the method described above can be used.

In the present invention, any monomer can be used as long as it is copolymerizable with VLA. Particularly, monomers described below are advantageously used among others.

Since VLA is a styrene derivative, a styrenic monomer having the same polymerization group is advantageously used from the viewpoint of copolymerizability. Specifically, an example of the monomer is represented by the following formula (5).

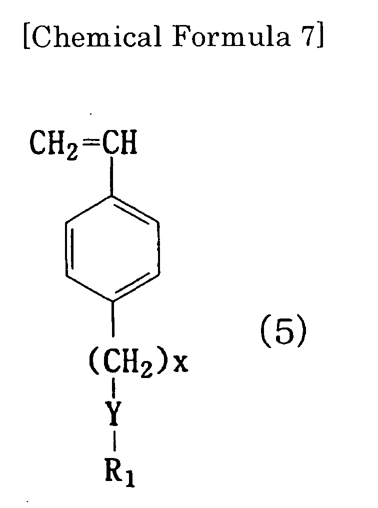

[wherein x represents an integer of 0 to 3, -Y- is a single bond or an atomic group represented by -NHCO-, NHCONH-, -CONH-, -OCONH-, -NHCOO-, -OCO-, -COO-, -CO- or -NH-, and further, R₁ is any one of the following (a) and (b) :
(a) a saturated or unsaturated i) monocycle, ii) bicycle or iii) five-membered or six-membered heterocycle containing O atom, S atom or N atom, which has 5 to 10 carbon atoms, wherein the monocycle, bicycle, five-membered or six-membered heterocycle may be at least partly substituted by at least one selected from the group consisting of a halogen atom, a hydroxy group, an amino group, a thiol group, a cyano group, a cyclopentyl group, a cyclohexyl group, a phenyl group and an acetyl group; and
(b) an alkyl group having carbon atoms of 1 to 20 or an atomic group represented by general formula: -O-C_{α}H_{2α+1}, -S-C_{α}H_{2α+1} or -NH-C_{α}H_{2α+1} (in which α represents an integer of 1 to 20), wherein the alkyl group or atomic group may be at least partially substituted by at least one selected from a halogen atom, a hydroxy group, an amino group, a thiol group, a cyano group, a cyclopentyl group, a cyclohexyl group, a phenyl group and an acetyl group.]

Of such monomers, monomer (5a) represented by the following formula (5a) [vinylbenzylhexadecanamide] and monomer (5b) represented by the following formula (5b) [vinylbenzylcyclohexylpropanamide] are particularly advantageously used. When the contact lens solution containing the copolymer of each of these monomers and VLA is used as a preservative solution, such a copolymer is well adsorbed into the surface of the contact lens to improve water wettability of the surface of the contact lens (to impart hydrophilicity to the surface of the contact lens) and to effectively maintain excellent water wettability (hydrophilicity) thereof. According to the finding of the present inventors, the contact lens solution containing the copolymer of the above-mentioned monomer and VLA exhibits the excellent effect particularly to silicone-based contact lenses.

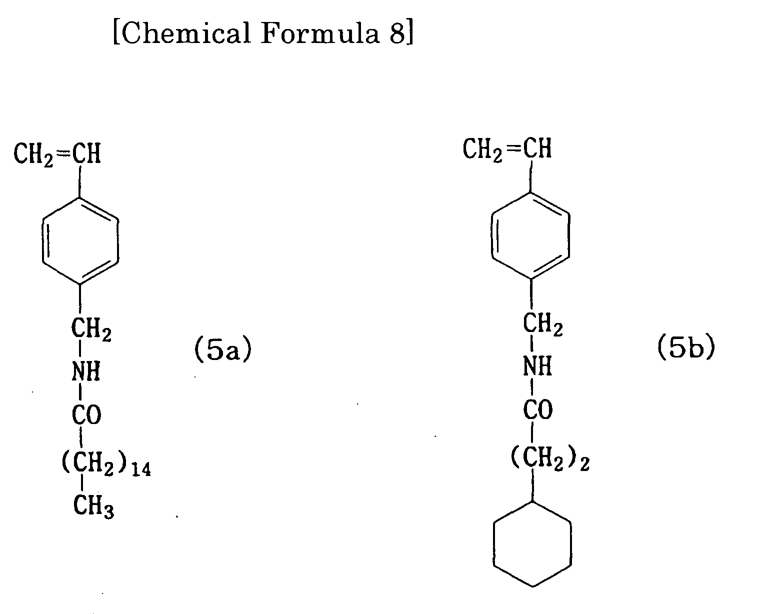

Further, additional examples of monomers which are easily copolymerized with VLA include vinyl group-containing monomers. Specifically, examples of the vinyl group-containing monomer include vinyl carboxylate monomers such as vinyl acetate, vinyl propionate and vinyl stearate, N-vinyl lactams such as N-vinyl-2-piperidone, N-vinyl-2-pyrrolidone, N-vinyl-2-caprolactam, N-vinyl-3-methyl-2-pyrrolidone, N-vinyl-3-methyl-2-piperidone, N-vinyl-3-methyl-2-caprolactam, N-vinyl-4-methyl-2-pyrrolidone, N-vinyl-4-methyl-2-piperidone, N-vinyl-4-methyl-2-caprolactam, N-vinyl-5-methyl-2-pyrrolidone, N-vinyl-5-methyl-2-piperidone and N-vinyl-3-ethyl-2-pyrrolidone, vinylcyclopentane, and further a monomer represented by the following formula (6):

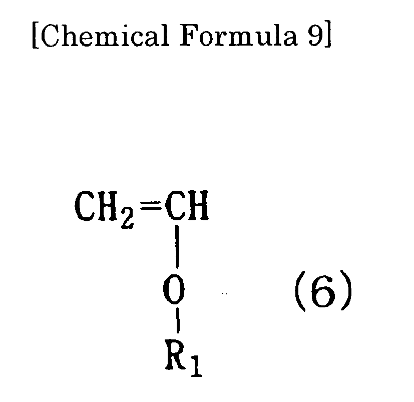

[wherein R₁ has the same meaning as in formula (5).]

Of these, vinylcyclopentane, monomer (6a) represented by the following formula (6a) [ethylene glycol monovinyl ether] and monomer (6b) represented by the following formula (6b) [diethylene glycol monovinyl ether] are particularly advantageously used.

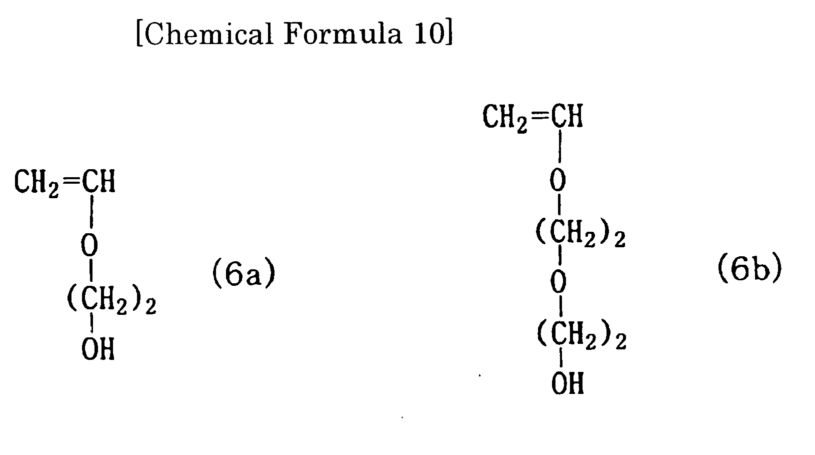

Further, in recent years, the majority of the contact lenses to which hydrophilization treatment is performed by using the contact lens solution of the present invention contain a siloxane compound as a constituent component to improve oxygen permeability. From the viewpoint of affinity with such contact lenses containing the siloxane compound as the constituent component, in the present invention, a silicon-based monomer represented by the following formula (7) is advantageously used as the monomer copolymerizable with VLA.

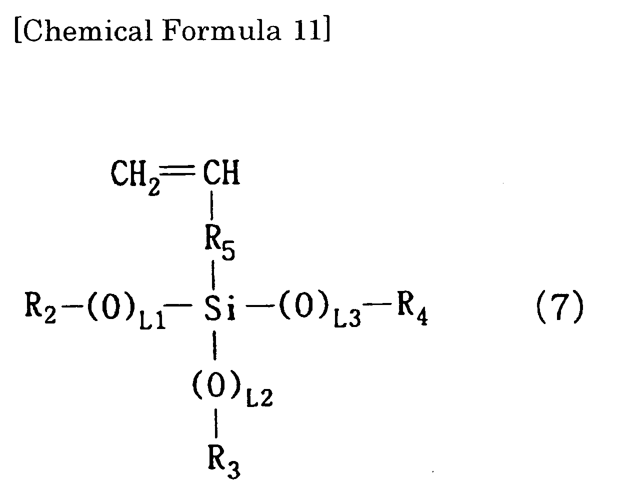

[wherein L1, L2 and L3 are each independently 0 or 1; and R₂, R₃ and R₄, which are independent from one another, are each an alkyl group having 1 to 20 carbon atoms or an atomic group represented by general formula: -O-C_{β}H_{2β+1}, -S-C_{β}H_{2β+1} or -NH-C_{β}H_{2β+1} (in which β represents an integer of 1 to 20), wherein the alkyl group or atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group; further, -R₅- is any one of the following (a) and (b) :
(a) an atomic group represented by a single bond, or an atomic group represented by -NHCO-, -NHCONH-, -CONH-, -OCONH-, -NHCOO-, -OCO-, -COO-, -CO-, -NH- or -O-; and
(b) an atomic group represented by general formula: -C_{γ}H_{2γ}-, -O-C_{γ}H_{2γ}-, -C_{γ}H_{2γ}-O-, -S-C_{γ}H_{2γ}-, -C_{γ}H_{2γ}-S-, -NH-C_{γ}H_{2γ}- or -C_{γ}H_{2γ}-NH- (in which γ represents an integer of 1 to 20), wherein the atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group.]

Of such silicon-based monomers, ones represented by the following formulas (7a) to (7c) are particularly advantageously used.

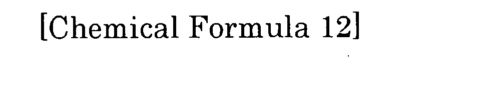

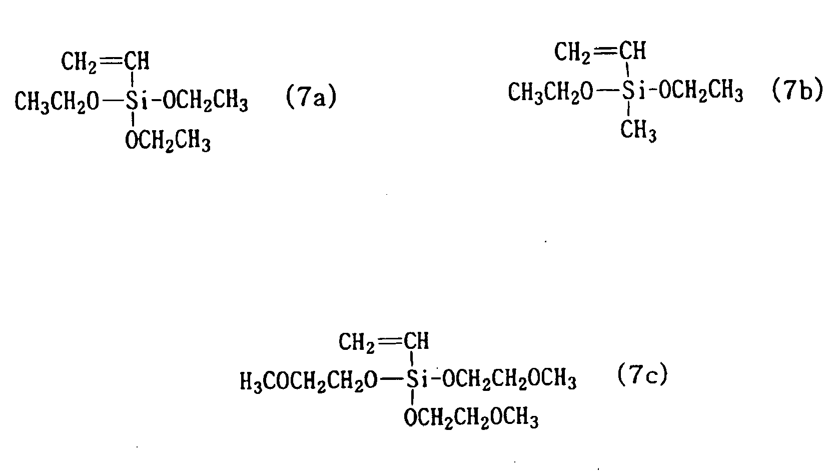

Copolymerization of the above-mentioned monomer and VLA is performed, for example, as follows. That is to say, according to characteristics required for the contact lens solution as a final object, one or more monomers are selected from the monomers copolymerizable with VLA, including the above-mentioned monomers, and such one or more monomers and VLA are mixed with each other. This mixture is dissolved in a solvent such as water, tetrahydrofuran or dimethyl sulfoxide (DMSO), as needed. Then, a polymerization initiator is added to perform radical polymerization, thereby being able to obtain the desired copolymer (hereinafter also appropriately referred to as the VLA copolymer).

As the polymerization initiator used in performing the copolymerization, any one can be used as long as it has hitherto been widely used. Examples thereof include benzoyl peroxide, lauroyl peroxide, diisopropyl peroxydicarbonate, t-butylperoxy-2-ethylhexanoate, t-butylperoxydiisobutylate, azobisisobutyronitrile, azobisisodimethylvaleronitrile, persulfates, and persulfate-bisulfite systems.

Further, various conditions in performing the copolymerization, such as the amount of VAL and the monomer used are appropriately determined according to the kind of monomer and the desired copolymer. For example, in the above-mentioned silicon-based monomer, when the copolymerization ratio thereof is too large, there is a risk of deterioration in copolymerizability or a risk of deterioration in water solubility of the resulting copolymer. Accordingly, a copolymerization ratio that does not cause such a problem is employed.

As the copolymer contained in the contact lens solution of the present invention, one having a weight average molecular weight of about 10000 to 2000000 is preferably used to provide solubility in the solution and to effectively impart hydrophilicity to the contact lens. When the weight average molecular weight is too small, the copolymer is poor in a continuing effect of hydrophilicity in some cases. In the copolymer having such a large weight average molecular weight as exceeding 2000000 polymerization or purification thereof becomes difficult.

Further, the concentration of the copolymer in the contact lens solution of the present invention is appropriately determined according to the purpose of use of such a solution (for example, use as a circulation preservative solution, use as a washing preservative solution, or the like), the mode of use (daily use, periodical use, or the like), the state of use (momentary contact with a lens in washing, overnight immersion of a lens therein, or the like), the kind of intended contact lens, or the like. In general, the copolymer is contained in the solution in an amount of 0.0001 to 5 w/v%. It is preferable to be contained in an amount of 0.001 to 2.0 w/v%, more preferably 0.01 to 0.5 w/v%. When the concentration of the copolymer is less than 0.0001 w/v%, there is a risk of failing to sufficiently achieve hydrophilization of the surface of the contact lens. On the other hand, even when the copolymer is used at a concentration higher than 5 w/v%, a hydrophilizing effect is not enhanced so much, and a waste of the copolymer due to heavy use thereof increases.

A mechanism of imparting hydrophilicity to a contact lens by the specified copolymer in the contact lens solution of the present invention is not completely elucidated yet by the present inventors. However, the present inventors presume that polystyrene chains contained in a main chain skeleton of the copolymer and side chains of the monomer copolymerized with VLA are linked by hydrophobic bonds to the hydrophobic surface of the contact lens, resulting in formation of a surface layer of the lens by hydrophilic lactose as side chains of VLA. In particular, in conventional contact lens solutions, saccharides are used as it is, so that bond to the surface of the contact lens having hydrophobicity is not sufficient and easily separated by rinsing with city water or the like. As a result, there has been a problem in continuousness or durability of the hydrophilizing effect. However, in the contact lens solution of the present invention, the hydrophobic bonds between the copolymer contained therein and the surface of the contact lens are relatively strong, so that the hydrophilizing effect can be effectively maintained. Another advantage of using the specified copolymer is that the copolymer has a hydrophilic moiety and a hydrophobic moiety at once, so that a surface active effect also can be expected. That is to say, it is possible to use the contact lens solution of the present invention not only as the preservative solution of contact lenses, but also as a washing agent.

In order to effectively remove stains such as eye mucus adhered to contact lenses, a surfactant is advantageously contained in the contact lens solution of the present invention. As such a surfactant, any one can be used as long as it has hitherto been used in a contact lens solution. However, the surfactant is appropriately selected and used according to the kind of contact lens to be treated with the solution, and the like.

For example, for the solution for hydrated soft contact lens, it is desirable that the surfactant has no influence on lens materials, not to mention having safety, and also having no influence on its disinfection effect, taking into account the case where this solution is used as a multipurpose solution. From such a viewpoint, a disinfectant used in the multipurpose solution is generally cationic, so that an anionic surfactant is anticipated to bind thereto to decrease the disinfection effect. A cationic surfactant and an amphoteric surfactant are particularly strong in interaction with the hydrated soft contact lenses and tend to be adsorbed into the lens materials. Accordingly, in the end, a nonionic surfactant is preferred. Incidentally, the multipurpose solution is becoming a mainstream as a care solution for the recent hydrated soft contact lenses, and generally means a solution for performing treatments such as washing, preservation and disinfection by a single solution.

Examples of such nonionic surfactants include polyoxyethylene-polyoxypropylene glycol copolymers, polyoxyethylene-polyoxypropylene-substituted ethylenediamine, glycerin fatty acid esters, sucrose alkyl esters, polyoxyethylene alkylamines, polyoxyethylene sorbitan fatty acid esters, triethanolamine fatty acid esters, polyoxyethylene alkyl ethers, polyoxyethylene alkyl phenyl ethers, polyoxyethylene hydrogenated castor oil. Of these, the polyoxyethylene-polyoxypropylene glycol copolymers, polyoxyethylene-polyoxypropylene-substituted ethylenediamine and polyoxyethylene hydrogenated castor oil are advantageously used because of their high safety to living organisms. The concentration of such nonionic surfactants is appropriately determined according to the amount of the VAL copolymer in the solution, and the like. When the concentration is too low, there is a risk of failing to sufficiently obtain the effect by blending. On the other hand, when the concentration is too high, there is a risk of inhibiting the functions of the copolymer and other components in the solution. Accordingly, in the contact lens solution of the present invention, the nonionic surfactant is allowed to be contained generally in an amount of 0.001 to 10 w/v%, preferably 0.01 to 3 w/v%.

In contrast, for the care solution for hard type contact lenses, an antiseptic effect of the solution is generally required, but there is little request for positively disinfecting the lenses. Hydrophobicity of surfaces of the lenses is strong, so that lipid stains are liable to rather adhere thereto. Accordingly, the surfactant having a high effect of removing such stain is advantageously blended. Specifically, an anionic surfactant, an amphoteric surfactant, a cationic surfactant or the like is appropriately selected.

As the anionic surfactant, there can be used, for example, an alkyl sulfate, an alkylbenzene sulfonate, an N-acyltaurine salt, an α-olefin sulfonate, a polyoxyethylene alkyl ether phosphate, a polyoxyethylene alkyl ether sulfate, a polyoxyethylene alkyl phenyl ether sulfate, a di(polyoxyethylene alkyl ether) phosphate. Of these, a sodium alkyl sulfate, a sodium alkylbenzene sulfonate, a sodium α-olefin sulfonate, a sodium polyoxyethylene alkyl ether sulfate and a sodium polyoxyethylene alkyl phenyl ether sulfate have been reported to exhibit an excellent washing effect and to exhibit an excellent effect even in brief immersion preservation when it is used in combination with the nonionic surfactant.

Further, examples of the amphoteric surfactants include glycine type surfactants such as lauryldiaminoethylglycine, acetic acid betaine type surfactants such as lauryldimethylaminoacetic acid betaine, imidazolium betaine type surfactants such as a 2-alkyl-N-carboxymethyl-N-hydroxyethyl imidazolium betaine, tertiary aminoxides such as an alkyl dimethyl aminoxide. Further, examples of the cationic surfactants include octadecylamine acetate, octadecyl ammonium chloride, alkyl dimethyl benzyl ammonium chloride.

Since the amount of such surfactants depends on the kind of surfactant to be selected or to be combined, it is not necessarily, but generally the amphoteric surfactant is contained in an amount of 0.001 to 10 w/v%, preferably 0.01 to 3 w/v%, as with the above-mentioned nonionic surfactant.

In addition to the components as described above, one or two or more kinds of various additive components which have hitherto been used in the contact lens solutions may be further added to the contact lens solution of the present invention within the ordinary range, as needed.

For example, a buffer can be added to the contact lens solution of the present invention as a component for stabilizing the pH to improve preservation stability of the respective components contained in the solution, or for preventing the eye from smarting, even when it gets in the eye in wearing the contact lens. As such a buffer, an appropriate one can be selected and used from buffers generally used in the contact lens solutions. Specific examples thereof include boric acid buffers, phosphoric acid buffers, citric acid buffers, acetic acid buffers, glycine buffers, and tris buffers. The compounding ratio of such buffers is desirably from about 0.1 to 10 w/v%, and preferably from 0.2 to 5 w/v%. When the compounding ratio thereof is too small, there is a risk of failing to exhibit an effect for stabilizing the pH. On the other hand, when compounding ratio is too large, the amounts of the other components added relatively decrease to cause a risk of exerting adverse effects on exhibitions of effects by the other components.

Further, an isotonizing agent can be added to the contact lens solution of the present invention, for the purpose of adjusting the osmotic pressure in order to eliminate irritation to the eye. In particular, when the contact lens is worn on the eye in a state where the preservative solution is retained in the lens material, as in the case of the hydrated soft contact lens, it is necessary to adjust the osmotic pressure of the contact lens solution to a state close to the osmotic pressure of the tear liquid (280 to 300 mOs/kg). Specific examples of the isotonizing agents which have hitherto been used include ophthalmic physiologically acceptable inorganic salts such as sodium chloride and potassium chloride, the buffers described above and the like. Further, propylene glycol, glycerin or the like as a nonionic isotonizing agent can be also used in the contact lens solution of the invention. Such isotonizing agents are added preferably at such a quantitative ratio that the concentration thereof in the solution becomes about 0.01 to 0.5 mol/L, more preferably 0.05 to 0.15 mol/L. When the concentration is less than 0.01 mol/L, the osmotic pressure decreases. On the other hand, when the concentration exceeds 0.5 mol/L, the osmotic pressure excessively increases, which may cause a risk of giving irritation to the eye in wearing the lens. Incidentally, when the lens is once rinsed with city water after immersed in the contact lens solution of the invention, and then worn, as in the case of the unhydrated contact lens, it is unnecessary to particularly pay attention to the adjustment of the osmotic pressure.

Furthermore, a thickener can be contained to improve slippage of the lens at the time of washing with the fingers. Examples of thickeners include polyvinyl alcohol, polyvinyl pyrrolidone, carboxymethylcellulose, hydroxypropyl methylcellulose, gum arabic, polysaccharides and salts thereof. One or two or more kinds of thickeners selected from the above are added to the solution at such a quantitative ratio that the purpose of addition thereof is sufficiently achieved.

In addition, a disinfectant can be added so that bacterial contamination of the contact lens solution is prevented, or so that the contact lens can be positively disinfected. Specific examples of such disinfectants include sorbic acid, sodium benzoate, benzalkonium chloride, chlorhexidine, pyridinium bromide, chlorobutanol, polyhexamethylene biguanide hydrochloride, benzyl alcohol, and polyquaterium. Although the amount of these disinfectants added varies depending on the kind of disinfectant used, an amount enough to kill bacteria, fungi and the like and inhibit reproduction thereof is sufficient. Usually, it is adjusted so that the concentration thereof becomes about 0.0001 to 0.5 w/v%.

In addition to the above, various additives may be further used, depending on specific use. For example, it is also possible to incorporate an enzyme for removing protein stains adhered to the contact lens, to add a chelating agent for preventing deposition of calcium and the like in the tear liquid adhered to the contact lens, or to use a refreshing agent together for the purpose of providing a refreshing feeling at the time of wearing contact lens after immersion. Examples of the chelating agents include ethylenediaminetetraacetic acid and sodium salt thereof. Examples of refreshing agent include menthol, camphor, borneol, geraniol, eucalyptus oil, bergamot oil, fennel oil, mentha oil, rose oil, and coolmint. These additive components are each added at a quantitative ratio according to the purpose of addition thereof.

By the way, the contact lens solution of the present invention is prepared by adding and incorporating the respective components as described above in respective proper amounts into an appropriate aqueous medium in a manner similar to the conventional one. As the aqueous medium used on that occasion, any solution can be employed in addition to water itself such as city water, purified water or distilled water, as long as it is a solution mainly composed of water, high in safety to living organisms and ophthalmically sufficiently allowable. Further, in preparing such a contact lens solution, no particular method is required at all. It can be easily obtained by dissolving the respective components in the aqueous medium in any order in the same manner as preparation of ordinary aqueous solution.

Then, when hydrophilizing treatment is performed to the contact lens by using the contact lens solution thus prepared as a hydrophilizing agent, it is only required to contact the contact lens solution with the contact lens. Further, it is also effective to immerse the contact lens in the contact lens solution, and then, rinse the contact lens taken out from the solution with city water or the like.

That is to say, when the contact lens solution of the present invention is contacted with the contact lens, the VLA copolymer in the solution is adsorbed into the surface of the lens to constitute an adsorption layer, and the presence of this adsorption layer effectively hydrophilizes the surface of the lens. Moreover, such an adsorption layer is not easily removed after the formation thereof on the surface of the lens even when rinsed with water, so that the surface of the lens is hydrophilized extremely well by a simple and easy operation.

Further, hydrophilicity is continuously maintained by preserving the contact lens which is removed after being worn in the contact lens solution of the present invention. Surfaces of the conventional hydrated contact lenses are essentially hydrophilic, so that the significance of venturing to apply the solution of the present invention is small. However, in many recent low-hydrated, high-oxygen-permeable soft contact lenses, silicon- or fluorine-containing monomers such as siloxanyl (meth)acrylates, polydimethylsiloxane macromers or fluoroalkyl (meth)acrylates are used. Accordingly, some lenses do not have good water wettability of the surfaces thereof, even if they are the hydrated soft contact lenses. The contact lens solution of the present application is worthy to apply to such hydrated soft contact lenses poor in water wettability. To the above-mentioned low-hydrated, high-oxygen-permeable soft contact lenses, the contact lens solution containing the copolymer of VLA and the silicon-based monomer is advantageously used.

Incidentally, since these high-oxygen-permeable soft contact lenses can be worn for a long-time as might be expected, it is difficult to directly perform treatment such as recovery of hydrophilicity of the contact lenses, between the time when the lenses are taken out from the preservative solution or the like and the time when the lenses are immersed in the preservative solution or the like again and preserved. Even in such a case, the VLA copolymer contained in the contact lens solution of the present invention can be selected, taking into account affinity with the lens material, which makes it possible to continue good hydrophilicity of the surfaces of the contact lenses for a long period of time.

### EXAMPLES

To further clarify he present invention, some examples of the present invention will be described. It is to be understood that the present invention is not limited to the details of the description of such examples, but may be embodied with various changes, modifications and improvements that may occur to those skilled in the art, without departing from the scope of the invention.

Initially, N-p-vinylbenzyl-D-lactonamide (VLA) was synthesized according to the following procedure.

### -Synthesis of VLA-

In a recovery flask having a specified volume, 1 kg of p-vinylbenzyl chloride and 1.2 kg of phthalimide potassium were dissolved in 3.2 L of N,N-dimethylformamide (DMF), and then, the solution was heated at 50°C for 4 hours. Then, DMF was removed by distillation from the solution using an evaporator, and thereafter, 4.5 L of benzene was added to dissolve the residue. The resulting benzene solution was washed several times with a 0.2 normal (N) of NaOH aqueous solution (the total amount of the NaOH aqueous solution: 3.25 L), and further washed several times with water (the total amount of water used: 3.25 L). The benzene solution after such washing was dried by using sodium sulfate, and then, the solvent was removed by distillation using an evaporator. Thereafter, the resulting residue was recrystallized using methanol, thereby obtaining 1.5 kg of vinylbenzylphthalimide.

In a separable flask having a specified volume, 1 kg of vinylbenzylphthalimide thus obtained was dissolved in 2.7 L of ethanol, and the resulting ethanol solution was heat-refluxed under a nitrogen stream. A solution obtained by dissolving 0.36 kg of 80% hydrazine monohydrate in 545 mL of ethanol was added dropwise into such a separable flask using a dropping funnel for about 40 minutes. The reaction of vinylbenzylphthalimide with hydrazine monohydrate was conducted with heat-refluxing for 90 minutes from the start of dropping of the solution. Then, solid matter obtained by the reaction was taken by filtration, and a KOH solution (a solution in which 1 kg of KOH was dissolved in 6.5 L of water) was added thereto to dissolve the solid matter. Then, the KOH solution in which such solid matter was dissolved was extracted with ether. For such extraction, 3.6 L of ether was used per one time, and the extraction was carried out total of three times. An ether layer obtained by the extraction was fractionated, and such an ether layer (ether solution) was washed with a 2% potassium carbonate aqueous solution, and further washed several times with water. After the ether solution being washed was dried by using sodium sulfate, ether was removed by distillation, and the residue was distilled under reduced pressure, thereby obtaining 0.45 kg of vinylbenzylamine. Vinylbenzylamine thus obtained was used not only in synthesis of VLA, but also in synthesis of a monomer copolymerized with VLA, as described later.

Meanwhile, 12 g of lactose was dispersed in 300 mL of methanol, followed by heating to 40°C. A methanol solution obtained by dissolving 18 g of iodine in 15 ml of methanol was added dropwise to this dispersion solution, followed by reaction for 40 minutes. A 4N of KOH methanol solution was added dropwise to such a reaction solution until the color of iodine disappeared from the reaction solution. Thereafter, a precipitate developed in the reaction solution was separated by filtration, and the precipitate separated by filtration was washed several times with cold methanol to obtain crystals of a reaction product. The resulting crystals were once weighed. The resulting crystals were dissolved in a tiny amount of water, and an acidic fraction was isolated from this aqueous solution by using a proton type ion exchange resin (trade name: AMBERLITE IR120B, manufactured by Rohm and Haas Company, USA). Methanol and ethanol were added to such an aqueous solution after isolation, followed by dehydration, and the resulting solid matter was completely dried. This operation was repeated several times to obtain desired lactose lactone.

In an eggplant-shaped flask having a specified volume, 1 kg of lactose lactone obtained as described above was dissolved in 5.4 L of methanol at 70°C, and then, 0.4 kg of vinylbenzylamine described above was added to this methanol solution, followed by reaction at 70°C for 120 minutes. After the termination of the reaction, 21.7 L of acetone was added to the recovery flask to precipitate a reaction product (VLA). After allowed to stand at 4°C for several hours, the precipitate was taken by filtration, and recrystallized using methanol. The yield of VLA recrystallized was 1.1 kg.

Meanwhile, monomers to be copolymerized with VLA were each made ready as described below.

### -Synthesis of Monomer (5a)-

The above-mentioned monomer (5a) [vinylbenzylhexadecanamide] was synthesized in the following manner. The previously obtained 10 g of vinylbenzylamine was dissolved in 150 mL of anhydrous chloroform, and 7 g of pyridine was further added thereto and the solution was cooled. After such cooling, 50 mL of anhydrous chloroform in which 24 g of palmitoyl chloride was dissolved was added dropwise to the solution using a dropping funnel for 30 minutes. After the termination of dropping, the chloroform solution was washed with water, and dried. Thereafter, chloroform was distilled off under reduced pressure. Then, the precipitate developed by distillation under reduced pressure was recrystallized using ethanol, thereby obtaining 20 g of monomer (5a).

### -Synthesis of Monomer (5b)-

The above-mentioned monomer (5b) [vinylbenzylcyclohexylpropanamide] was synthesized in the following manner. The previously obtained 13 g of vinylbenzylamine was dissolved in 150 mL of anhydrous tetrahydrofuran (THF), and 50 mL of anhydrous THF in which 23 g of 1-ethyl-3-(3-dimethylaminopropyl)carbodiimide (a water-soluble carbodiimide, WSC) and 13 g of 3-cyclohexylpropionic acid were dissolved was further added. After the termination of the reaction, a precipitate (reaction product) was separated by filtration, and THF was distilled off from the precipitate separated by filtration. The reaction product thus obtained was dissolved in chloroform, and the chloroform solution was washed with water, and dried. Thereafter, chloroform was distilled off under reduced pressure. Then, the precipitate developed by distillation under reduced pressure was recrystallized using ethanol, thereby obtaining 10 g of monomer (5b).

Ethylene glycol monovinyl ether (TCI product code: E0518) manufactured by TOKYO CHEMICAL INDUSTRY CO., LTD. was provided as monomer (6a), and diethylene glycol monovinyl ether (TCI product code: D2623) manufactured by the above company was provided as monomer (6b).

As monomers (A) to (F) having structures shown below, commercially available products were also provided, respectively.

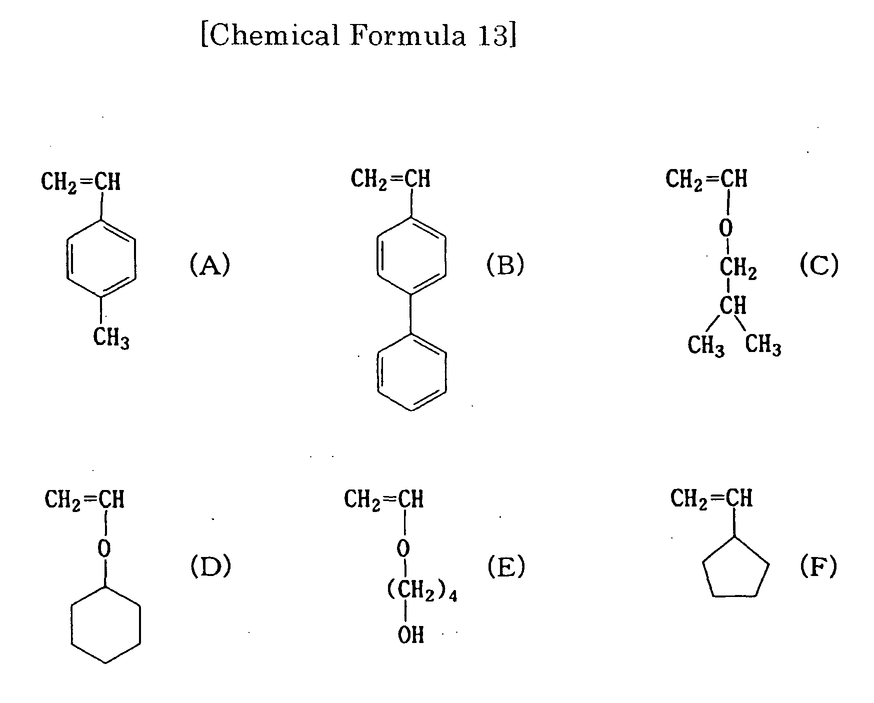

As the above-mentioned silicone-based monomers [monomers (7a) to (7c)], commercially available products were provided, respectively. Just to make sure, structures of such monomers are shown below:

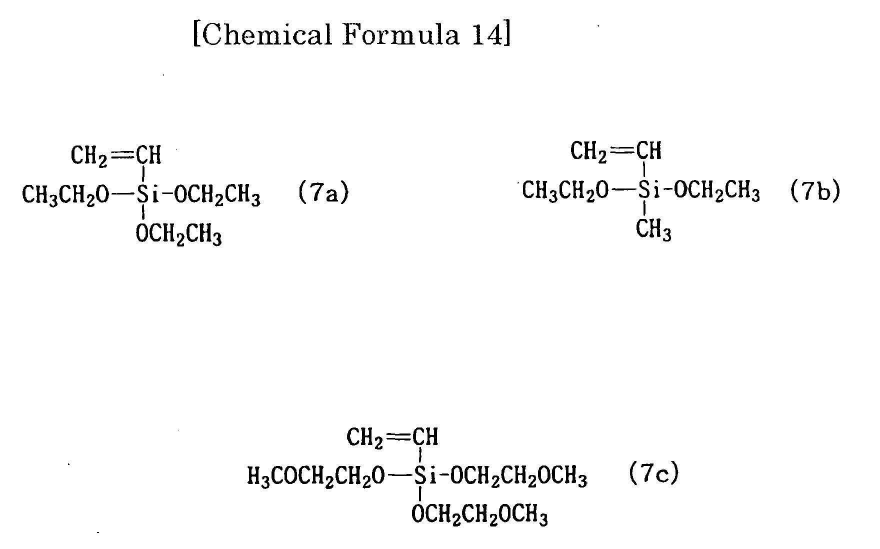

Using N-p-vinylbenzyl-D-lactonamide (VLA) and the respective monomers provided as described above, a plurality of copolymers were synthesized. Specifically, first, VLA and any one of the respective monomers were weighed so as to be a molar ratio shown in the following Table 1 or 2, and separately poured into a vacuum reaction tube. Then, dimethyl sulfoxide (DMSO) or a mixed solvent of DMSO and toluene was added into the reaction tube as a solvent, and copolymerization components (VLA and the monomer) were completely dissolved. Thereafter, a process of freezing-degassing-thawing defined as one cycle was repeated plural times for the solution to remove oxygen in the solution. Then, 0.01 mol of azoisobutyronitrile (AIBN) was added to the solution and dissolved, and thereafter, the inside of the vacuum reaction tube was sealed under reduced pressure. Then, the inside of the reaction tube was heated to 60°C to allow the copolymerization components to react for 5 hours.

**[Table 1]**

| | | Copolymer No. | | | | | | | | | |
|---|---|---|---|---|---|---|---|---|---|---|---|
| | | 1 | 2 | 3 | 4 | 5 | 6 | 7 | 8 | 9 | 10 |
| Copolymerization Composition (molar ratio) | VLA | 9 | 9 | 9 | 9 | 9 | 9 | 9 | 9 | 9 | 9 |
| | Monomer (5a) | 1 | - | - | - | - | - | - | - | - | - |
| | Monomer (5b) | - | 1 | - | - | - | - | - | - | - | - |
| | Monomer (6a) | - | - | 1 | - | - | - | - | - | - | - |
| | Monomer (6b) | - | - | - | 1 | - | - | - | - | - | - |
| | Monomer (A) | - | - | - | - | 1 | - | - | - | - | - |
| | Monomer (B) | - | - | - | - | - | 1 | - | - | - | - |
| | Monomer (C) | - | - | - | - | - | - | 1 | - | - | - |
| | Monomer (D) | - | - | - | - | - | - | - | 1 | - | - |
| | Monomer (E) | - | - | - | - | - | - | - | - | 1 | - |
| | Monomer (F) | - | - | - | - | - | - | - | - | - | 1 |

**[Table 2]**

| | | Copolymer No. | | | | |
|---|---|---|---|---|---|---|
| | | 11 | 12 | 13 | 14 | 15 |
| Copolymerization Composition (molar ratio) | VLA | 9 | 9 | 9 | 7 | 5 |
| | Monomer (7a) | 1 | - | - | - | - |
| | Monomer (7b) | - | 1 | - | - | - |
| | Monomer (7c) | - | - | 1 | 3 | 5 |

The solution after the reaction was added dropwise to a large excess of methanol to precipitate a reaction product. Then, this precipitate (reaction product) was dissolved in a specified solvent, and the resulting solution was added dropwise to methanol again to precipitate the reaction product. This treatment was repeated three times. When the monomer is slightly soluble in methanol, the reaction product after the above-mentioned treatment was washed several times with chloroform or toluene.

The reaction product to which such treatment was performed was dissolved in distilled water, and dialysis was performed to a large excess of water. In the dialysis, a cellulose membrane (molecular weight cutoff: 15000) manufactured by Sanko Junyaku Co., Ltd. was used. Then, the solution after the dialysis was lyophilized, thereby obtaining a desired copolymer (each of copolymer Nos. 1 to 15).

Using the resulting 15 kinds of copolymers, the following respective tests were conducted.

### < Contact Lens Water Wettability Evaluation Test I>

First, there was prepared an aqueous solution containing disodium hydrogenphosphate dodecahydrate in an amount of 0.37 w/v%, sodium dihydrogenphosphate dihydrate in an amount of 0.08 w/v% and sodium chloride in an amount of 0.83 w/v%. This aqueous solution had an osmotic pressure of about 290 (mOsm/kg·H₂O) and a pH of about 7.0. Copolymer No. 1 was dissolved in this aqueous solution to prepare two kinds of contact lens solutions (solutions Nos. 1 and 2) different in concentration of the copolymer No. 1. The solution having a copolymer in an amount of 0.05 w/v% is taken as solution No.1, and the solution having a copolymer in an amount of 0.1 w/v% is taken as solution No. 2.

One oxygen-permeable hard contact lens (trade name: DOCTOR'S EX-G. selling agency: EIKO Corporation) was immersed in 2 mL of each of these two kinds of contact lens solutions. After 10 minutes from the immersion, the lens was taken out from the solution, and lightly rinsed with city water. Thereafter, water wettability of a surface of the lens was visually observed. Meanwhile, as a control experiment, using the above-mentioned aqueous solution containing no polymer No. 1 (hereinafter referred to as aqueous solution α), the same treatment was performed to the oxygen-permeable hard contact lens, and the lens surface thereof was observed. The results of observation were compared to one another. As a result, it was confirmed that the contact lenses treated with the contact lens solutions according to the present invention (solutions Nos. 1 and 2) exhibited excellent water wettability.

Secondly, the following test was conducted to confirm in more detail that water wettability of the lens was improved by treating the contact lens with the contact lens solution of the present invention. The above-mentioned oxygen-permeable hard contact lenses were each immersed in 2 mL of each of the two kinds of contact lens solutions (solutions Nos. 1 and 2) and aqueous solution α for 10 minutes. After the immersion, the lens was taken out from the solution and air-dried. After such air-drying, the contact angle of each contact lens was measured. The measurement of the contact angle of the contact lens was conducted by adhering a drop of water to the lens and measuring the contact angle at a portion where the drop of water was in contact with the lens, from the side of the lens with a contact angle measuring instrument (goniometer type G-1, manufactured by Elma Kogaku Co., Ltd.).

As a result, the contact angle of the contact lens treated with solution No. 1 was 68°, and the contact angle of the contact lens treated with solution No. 2 was 63°. In contrast, the contact angle of the contact lens treated with solution α was 90°. From the measurement results, it was confirmed that water wettability of the lens was effectively improved when the contact lens was treated by the contact lens solution according to the present invention.

### <Contact Lens Wear Test I>

Initially, copolymer Nos. 1 to 10 was each dissolved in saline (NaCl concentration: 0.9 w/v%) to prepare 10 kinds of contact lens solutions (solution Nos. 3 to 12) each containing such a copolymer at a ratio of 0.05 w/v%. The copolymers used in the preparation of the respective contact lens solutions are shown in the following Table 3. Further, saline containing no copolymer was provided as a contact lens solution (solution No. 13).

**[Table 3]**

| Contact Lens Solution | Copolymer in Solution |
|---|---|
| Solution No. 3 | Copolymer No. 1 |
| Solution No. 4 | Copolymer No. 2 |
| Solution No. 5 | Copolymer No. 3 |
| Solution No. 6 | Copolymer No. 4 |
| Solution No. 7 | Copolymer No. 5 |
| Solution No. 8 | Copolymer No. 6 |
| Solution No. 9 | Copolymer No. 7 |
| Solution No. 10 | Copolymer No. 8 |
| Solution No. 11 | Copolymer No. 9 |
| Solution No. 12 | Copolymer No. 10 |
| Solution No. 13 | - |

One oxygen-permeable hard contact lens which was the same as used in the above-mentioned water wettability evaluation test I was immersed in 2 mL of each of these 11 kinds of contact lens solutions for 30 minutes, thereby treating a surface of the contact lens. Six test contact lenses were prepared by treating 6 contact lenses of one kind by using one kind of contact lens solution. Accordingly, since the 11 kinds of contact lens solutions are used, the total number of the test contact lenses prepared for this test is 66.

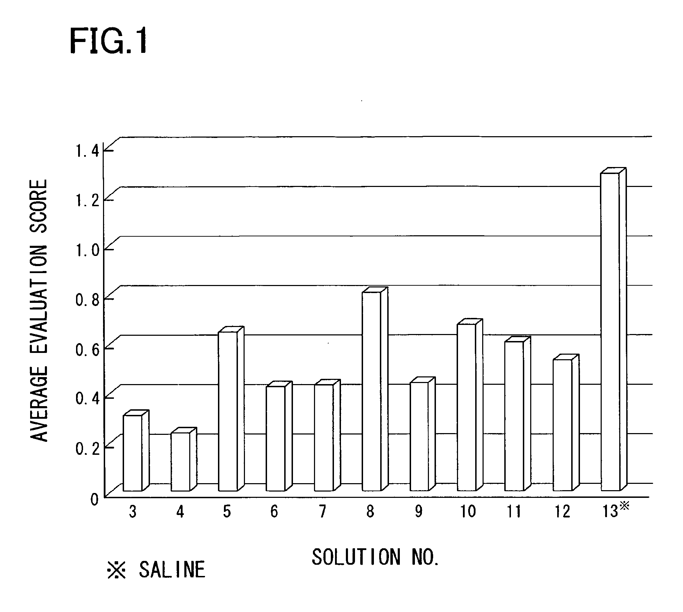

3 volunteers (test subjects) actually wore the contact lens, for 8 hours, which was taken out from the solution after being immersed in the solution for 30 minutes. Then, the test subjects evaluated the degree of cloudiness in the field of view which the test subjects themselves felt after the elapse of 8 hours, according to the evaluation criteria shown in the following Table 4. The evaluation scores of 3 volunteers were totaled up for each test contact lens treated with each solution, and the average evaluation score thereof was calculated. The results thereof are shown in Fig. 1 by a bar graph. As apparent from the evaluation criteria shown in the following Table 4, the lower the average evaluation score of the contact lens indicates that the volunteers felt less cloudiness in the field of view, even after wearing for 8 hours. In other words, in the contact lens having a low average evaluation score, it is shown that the solution used for treating the lens effectively inhibited the occurrence of cloudiness on the lens.

**[Table 4]**

| | |
|---|---|
| Evaluation Criteria | Evaluation Score |
| The field of view is clear. | 0 |
| The field of view is slightly clouded whitish. | 1 |
| No influence on eyesight, but the field of view is somewhat strongly clouded. | 2 |
| The field of view is strongly clouded to such a degree that an influence appears in eyesight. | 3 |

As apparent from the results shown in Fig. 1, it was confirmed that all the contact lens solutions according to the present invention (solution Nos. 3 to 12) could effectively inhibit the occurrence of cloudiness on the contact lenses. Above all, it was observed that the contact lens solution containing the copolymer No. 1 (solution No. 3) and the contact lens solution containing the copolymer No. 2 (solution No. 4) were particularly excellent.

### <Water Wettability Evaluation Test II of Contact Lens>

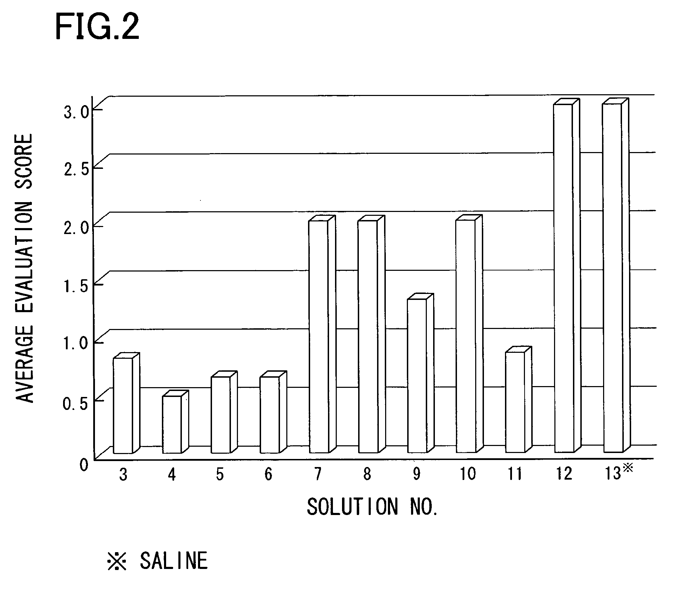

First, 11 kinds of contact lens solutions (solution Nos. 3 to 13) were prepared or provided in the same manner as in contact lens wear test I, and surfaces of the same oxygen-permeable hard contact lenses as above were treated by using such contact lens solutions, thereby providing 6 test contact lenses for each solution. The test contact lenses were taken out from the solutions, and strongly scrubbed with the fingers in running city water. After scrubbing, the degree of water wetting on surfaces of the contact lenses was visually observed, and evaluated according to the evaluation criteria shown in the following Table 5. The evaluation scores to 6 test contact lenses treated by using each solution were totaled up, and the average evaluation score thereof was calculated. The results thereof are shown in Fig. 2 by a bar graph. As apparent from the evaluation criteria shown in the following Table 5, the contact lens having the lower average evaluation score is more excellent in water wettability.

**[Table 5]**

| | |
|---|---|
| Evaluation Criteria | Evaluation Score |
| The whole surface of a lens is uniformly wet. | 0 |
| 90% or more of the whole surface of a lens is wet. | 1 |
| About 30 to 90% of the whole surface of a lens is wet. | 2 |
| Only less than 30% of the whole surface of a lens is wet. | 3 |

As apparent from the results shown in Fig. 2, it was confirmed that the contact lens solutions according to the present invention (solutions Nos. 3 to 12) imparted hydrophilicity to the contact lens to improve water wettability, although degrees of improvement differ from the solution. Above all, it was confirmed that solution Nos. 3 to 6 more effectively improved water wettability. Incidentally, in the above test, the test contact lenses taken out from the solutions are strongly scrubbed with the fingers in running city water. It is therefore considered that, for example, even solution No. 12 or the like in which great improvement in water wettability was not observed will give the sufficient effect, when used as a preservative solution for maintaining hydrophilicity of the surfaces of the contact lenses.

### <Contact Lens Water Wettability Evaluation Test III>

Copolymer No. 2 and each of surfactants were dissolved in water to prepare each of contact lens solutions (solution Nos. 14 to 18). As the surfactants, there were used a nonionic surfactant A (polyoxyethylene oleyl ether, trade name: EMULGEN 420, manufactured by Kao Corporation), a nonionic surfactant B (polyoxyethylene hydrogenated castor oil, trade name: NIKKOL HCO-60, manufactured by Nikko Chemicals Co., Ltd.), an amphoteric surfactant A (lauryldimethylaminoacetic acid betaine aqueous solution, manufactured by Nippon Oil and Fat Co., Ltd.), an amphoteric surfactant B (coconut oil fatty acid amidopropyldimethylaminoacetic acid betaine aqueous solution, trade name: Nikkol AM-3130N, manufactured by Nikko Chemicals Co., Ltd.) and an anionic surfactant (sodium polyoxyethylene lauryl ether sulfate aqueous solution, trade name: EMAL E-27C, manufactured by Kao Corporation). Further, the concentration of copolymer No. 2 in each solution, and the kind of surfactant contained and the concentration thereof are shown in the following Table 6. Furthermore, only each of the surfactants was dissolved in water to prepare each of contact lens solutions (solution Nos. 19 to 23). The surfactants used in the preparation are shown together in the following Table 6.

One oxygen-permeable contact lens (trade name: Q-1, manufactured by Polymer Technology Corporation) was immersed in 2 mL of each solution for 2 hours. After this immersion, the contact lens was taken out from the solution, and the lens taken out was rinsed with water for 10 seconds. Thereafter, the degree of water wettability on a surface of the contact lens was visually observed, and evaluated according to the evaluation criteria shown in the above-mentioned Table 5. The evaluation results (evaluation scores) thereof are also shown together in the following Table 6.

**[Table 6]**

| | Surfactant | Concentration (w/v%) | Concentration of Copolymer No. 2 (w/v%) | Evaluation Score |
|---|---|---|---|---|
| Solution No. 14 | Nonionic Surfactant A | 0.5 | 0.05 | 2 |
| Solution No. 15 | Nonionic Surfactant B | 0.5 | 0.05 | 2 |
| Solution No. 16 | Amphoteric Surfactant A | 1.5 | 0.075 | 0 |
| Solution No. 17 | Amphoteric Surfactant B | 1.5 | 0.075 | 0 |
| Solution No.18 | Anionic Surfactant | 0.5 | 0.05 | 2 |
| Solution No. 19 | Nonionic Surfactant A | 0.5 | - | 3 |
| Solution No. 20 | Nonionic Surfactant B | 0.5 | - | 3 |
| Solution No. 21 | Amphoteric Surfactant A | 1.5 | - | 3 |
| Solution No. 22 | Amphoteric Surfactant B | 1.5 | - | 3 |
| Solution No. 23 | Anionic Surfactant | 0.5 | - | 3 |

As apparent from the results shown in Table 6, it was confirmed that the contact lens solutions of the present invention further containing surfactant (solutions Nos. 14 to 18) imparted hydrophilicity to the contact lens to improve water wettability.

### < Contact Lens Water Wettability Evaluation Test IV>

Sodium hyaluronate (trade name: FCH-120, manufactured by Kibun Food Chemifa Co., Ltd.) was added to water, and dissolved therein. Trometamol (manufactured by Wako Pure Chemical Industries, Ltd., special grade chemical) and disodium ethylenediaminetetraacetate dihydrate (manufactured by Wako Pure Chemical Industries, Ltd.) were added thereto and dissolved. Thereafter, a polyhexamethylene biguanide solution (COSMOCIL CQ, manufactured by Arch Chemicals Japan, Inc.), an amphoteric surfactant (coconut oil fatty acid amidopropyldimethylaminoacetic acid betaine aqueous solution, trade name: Nikkol AM-3130N, manufactured by Nikko Chemicals Co., Ltd.) and copolymer No. 2 were further added and dissolved. To the solution, 3N hydrochloric acid was added in an appropriate amount, and water was further added, thereby preparing a contact lens solution (solution No. 24). Each of the obtained contact lens solution contained sodium hyaluronate in an amount of 0.1 w/v%, trometamol in an amount of 2.4 w/v%, disodium ethylenediaminetetraacetate dehydrate in an amount of 0.02 w/v%, the amphoteric surfactant in an amount of 1 w/v%, copolymer No. 2 in an amount of 0.05 w/v% and polyhexamethylene biguanide in an amount of 1 ppm, and had an osmotic pressure of 310 (mOsm/kg·H₂O) and a pH of 9.0. In addition, a contact lens solution (solution No. 25) containing all the above-mentioned components other than copolymer No. 2 in the same amount as in solution No. 24 was prepared.

One oxygen-permeable hard contact lens (trade name: Q-1) was immersed in 2 mL of each of the two kinds of thus prepared solutions for hours (immersion times) shown in the following Table 7. Then, the lens was taken out from the solution, and the degree of water wettability on a surface of the lens was visually observed and evaluated according to the evaluation criteria shown in the above-mentioned Table 5.

The term "Just after" in the columns of "Immersion Time" in the following Table 7 means "immediately after the contact lens was allowed to contact with the solution". Further, in the columns of "Evaluation Just after Taken Out", there are shown the results (evaluation scores) of observation and evaluation of the surface of the lens immediately after the contact lens was taken out from the solution. Furthermore, in the columns of "Evaluation after Water Rinsing", there are shown the results (evaluation scores) of observation and evaluation of the surface of the lens after the contact lens taken out from the solution was rinsed with water for 20 seconds. Moreover, in the columns of "Evaluation after Washing with Solution with Fingers", there are shown the results (evaluation scores) of observation and evaluation of the surface of the lens after the contact lens taken out from the solution was washed with the fingers by using the solution, and then, rinsed with water to remove foam and the like adhered to the surface of the lens.

**[Table 7]**

| | Immersion Time | Evaluation Just after Taken Out | Evaluation after Water Rinsing | Evaluation after Washing with Solution with Fingers |
|---|---|---|---|---|
| Solution No. 24 | Just after | 0 | 1 | 1 |
| | 3 Hours | 0 | 0 | 0 |
| | 24 Hours | 0 | 0 | 0 |
| Solution No. 25 | Just after | 1 | 3 | 3 |
| | 3 Hours | 0 | 3 | 3 |
| | 24 Hours | 0 | 3 | 3 |

As apparent from the results shown in Table 7, it was confirmed that solution No. 24 which was the contact lens solution according to the present invention and contained various components, which can be added to commonly used contact lens solutions, also imparted hydrophilicity to the contact lens to improve water wettability, compared to solution No. 25 containing no copolymer No. 2.

### <Contact Lens Wear Test II>

Initially, each one of copolymer Nos. 11 to 13 was dissolved in saline (NaCl concentration: 0.9 w/v%) to prepare 3 kinds of contact lens solutions (solution Nos. 26 to 28) each containing such a copolymer in an amount of 0.05 w/v%. The copolymers used for preparing the respective contact lens solutions are shown in the following Table 8. Further, like the above-mentioned contact lens wear test I, saline containing no copolymer was provided as a contact lens solution (solution No. 13). Further, as oxygen-permeable hard contact lenses, there were provided contact lens A (trade name: Q-1, manufactured by Polymer Technology Co., Ltd.), contact lens B (trade name: FP-60, manufactured by Paragon) and contact lens C (trade name: EM manufactured by Boston).

**[Table 8]**

| Contact Lens Solution | Copolymer in Solution |
|---|---|
| Solution No. 26 | Copolymer No. 11 |
| Solution No. 27 | Copolymer No. 12 |
| Solution No. 28 | Copolymer No. 13 |
| Solution No. 13 | - |

Each one of the 3 kinds of oxygen-permeable hard contact lenses was immersed in 2 mL of each of these 4 kinds of contact lens solutions (solution Nos. 13 and 26 to 28) for 30 minutes, thereby treating a surface of the contact lens. Six test contact lenses were prepared by treating 6 contact lenses of one kind by using one kind of contact lens solution. The 4 kinds of contact lens solutions and the 3 kinds of contact lenses were used, so that the total number of the test contact lenses prepared for this test was 72.

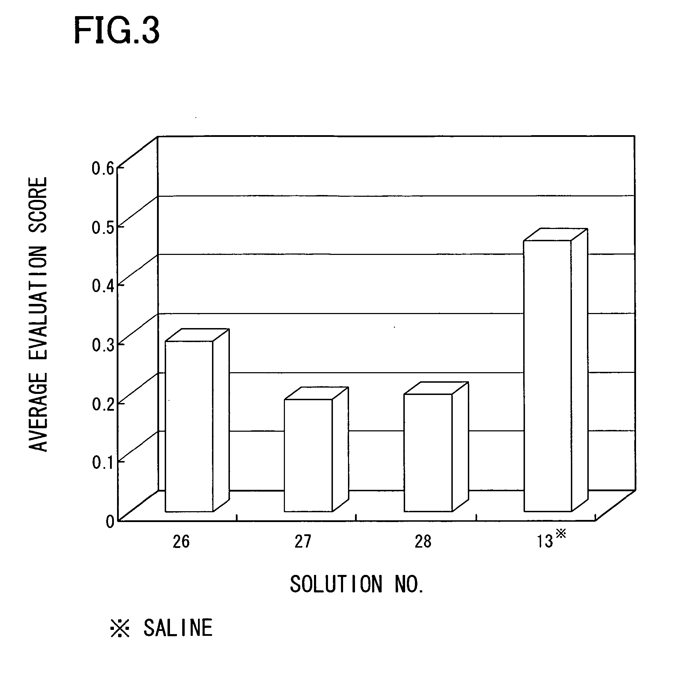

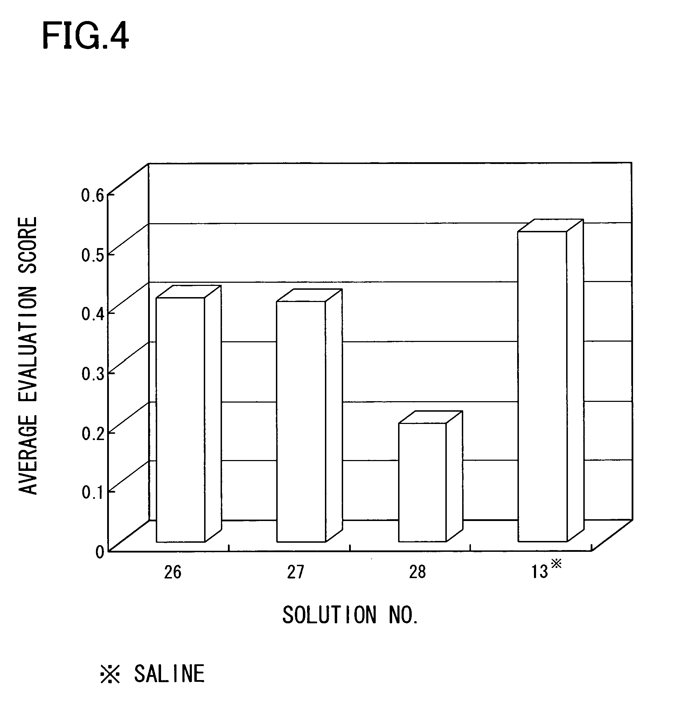

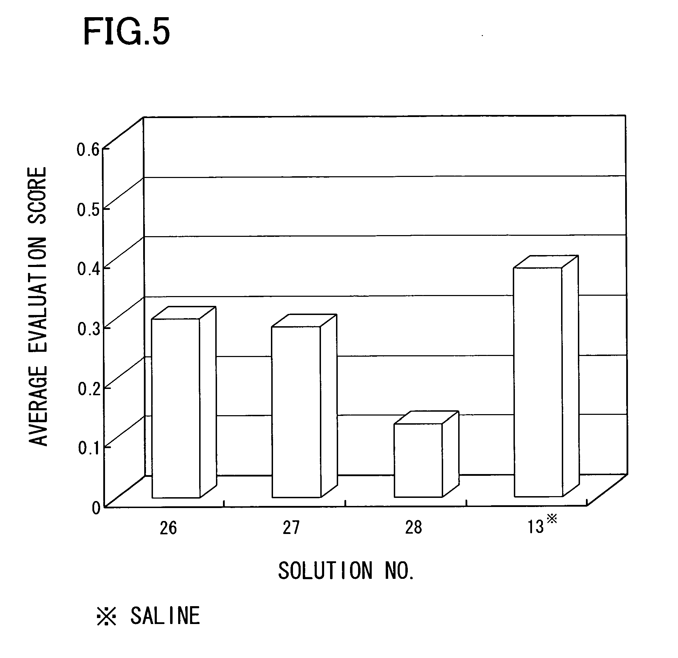

3 volunteers (test subjects) actually wore the contact lens, for 8 hours, which was taken out from the solution after being immersed in the solution for 30 minutes. Then, the test subjects evaluated the degree of cloudiness in the field of view which the test subjects themselves felt after the elapse of 8 hours, according to the evaluation criteria shown in the above Table 4. The evaluation scores of 3 volunteers were totaled up for each test contact lens, and the average evaluation score thereof was calculated. The average evaluation scores for the test contact lenses prepared by immersing contact lens A in the respective contact lens solutions are shown for each solution used for immersion by a bar graph in Fig. 3. Similarly, the average evaluation scores for the test contact lenses prepared by immersing contact lens B in the respective contact lens solutions are shown by a bar graph in Fig. 4, and the average evaluation scores for the test contact lenses prepared by immersing contact lens C in the respective contact lens solutions are shown by a bar graph in Fig. 5.

As apparent from the results shown in Figs. 3 to 5, it was confirmed that all the contact lens solutions according to the present invention (solution Nos. 26 to 28) effectively inhibited the occurrence of cloudiness on the contact lenses. Above all, it was observed that the contact lens solution containing copolymer No. 13 (solution No. 28) effectively inhibited the occurrence of cloudiness in all the 3 kinds of contact lenses.

### <Contact Lens Wear Test III>

Specified amount of Copolymer No. 13 was dissolved in saline to prepare 3 kinds of contact lens solutions (solution Nos. 29 to 31) different in concentration of the copolymer No. 13. The concentrations of copolymer No. 13 in the respective contact lens solutions are each shown in the following Table 9. Using the 3 kinds of contact lens solutions and 3 kinds of oxygen-permeable hard contact lenses (contact lenses A to C), a wear test same as the above-mentioned contact lens wear test II was conducted. The average evaluation score was calculated from evaluation scores indicated by 3 test subjects for each test contact lens. The results thereof are shown together in the following Table 9.

**[Table 9]**

| | | Solution No. 29 | Solution No. 30 | Solution No. 31 |
|---|---|---|---|---|
| | Concentration of Copolymer No. 13 [w/v%] | 0.01 | 0.025 | 0.05 |
| Average Evaluation Score | Contact Lens A* | 0.54 | 0.33 | 0.19 |
| | Contact Lens B* | 0.28 | 0.28 | 0.20 |
| | Contact Lens C* | 0.39 | 0.35 | 0.12 |

| | | | | |
|---|---|---|---|---|
| * The contact lens used in the test | | | | |

As apparent from the results shown in Table 9, it was confirmed that, depending on the kind of contact lens, the contact lens solution according to the present invention could effectively inhibit the occurrence of cloudiness, even when the solution have a low concentration of the specified copolymer that is an essential component. This shows that the adsorption mechanism of the copolymer, which is the essential component in the contact lens solution of the present invention, is largely related to the contact lens material. Accordingly, when the contact lens solution of the present invention is prepared, it is important to appropriately determine conditions such as the structure and the concentration of the copolymer according to the material of the contact lens for which the solution is used.

### <Contact Lens Wear Test IV>

Each one of copolymer Nos. 13 to 15 was dissolved in saline to prepare 3 kinds of contact lens solutions (solution Nos. 32 to 34) in which the copolymer is present in an amount of 0.1 w/v%. The copolymers contained in the respective contact lens solutions are each shown in the following Table 10. Using the 3 kinds of contact lens solutions and 3 kinds of oxygen-permeable hard contact lenses (contact lenses A to C), a wear test same as the above-mentioned contact lens wear test II was conducted. The average evaluation score was calculated from evaluation scores obtained by 3 test subjects for each test contact lens. The results thereof are shown together in the following Table 10.

**[Table 10]**

| | | Solution No. 32 | Solution No. 33 | Solution No. 34 |
|---|---|---|---|---|
| | Copolymer in Solution | Copolymer No. 13 | Copolymer No. 14 | Copolymer No. 15 |
| Average Evaluation Score | Contact Lens A* | 0.07 | 0.06 | 0.35 |
| | Contact Lens B* | 0.15 | 0.06 | 0.37 |
| | Contact Lens C* | 0.07 | 0.09 | 0.09 |

| | | | | |
|---|---|---|---|---|
| * The contact lens used in the test | | | | |

### <Water Retention Evaluation Test of Contact Lens>

Specified amount of Copolymer No. 14 was dissolved in saline to prepare a contact lens solution (solution No. 35) in which the copolymer No. 14 is present in an amount of 0.05 w/v%. Further, like the above-mentioned various tests, saline containing no copolymer was prepared as a contact lens solution (solution No. 13). Furthermore, as silicone hydrogel lenses subjected to the test, there were provided hydrous soft contact lens A (trade name: Acuvue Advance, manufactured by Johnson & Johnson K.K.) and hydrous soft contact lens B (trade name: O₂ OPTIX, manufactured by CIBA Vision Corporation).

Each one lens of the 2 kinds of hydrous soft contact lenses was immersed in 2 mL of each of the 2 kinds of contact lens solutions (solution Nos. 13 and 35) for 2 hours, thereby treating a surface of the contact lens. A total of 24 test contact lenses were prepared by treating 6 soft contact lenses of each kind by using each contact lens solution.

The test contact lens was taken out from the solution after being immersed for 2 hours. Then, a water layer (water film) on a surface of the lens was observed while the lens was hold by pinching an end thereof with tweezers. Then, the time from just after the test contact lens was taken out from the solution to when the water layer (water film) on the surface of the lens was broken was measured. The average value (sec) was calculated from the measurement results for 6 lenses of the same soft contact lenses treated with the same contact lens solution. The results thereof are shown in the following Table 11. The longer average value (sec) indicates that the lens surface has the higher water retention ability.

**[Table 11]**

| | Contact Lens Used in Test | Solution No. 35 | Solution No. 13* |
|---|---|---|---|
| Average Value [sec] | Hydrous Soft Contact Lens A | 59 | 30 |
| | Hydrous Soft Contact Lens B | 201 | 38 |

| | | | |
|---|---|---|---|
| * Saline | | | |

From such results shown in Table 11, it was confirmed that the contact lens solution according to the present invention could also contribute to improvement in water retention of the hydrous soft contact lens such as the silicone hydrogel lens.

### <Contact Lens Wear Test V>

Hydrous soft contact lens C (trade name: 2-Week Acuvue, manufactured by Johnson & Johnson K.K.) was provided as a disposable contact lens, together with the contact lens solutions (solution Nos. 13 and 35) and the silicon-containing hydrogel lenses (hydrous soft contact lenses A and B) used in the above-mentioned water retention test. Using these, as with the above-mentioned water retention test, a total of 36 test contact lenses were prepared by immersing 6 hydrous soft contact lenses of each kind in each contact lens solution for 2 hours.

The test contact lenses taken out from the solution after the elapse of 2 hours from the start of immersion were worn by 3 volunteers (subjects). Then, the subjects were forced to stop blinking for a while, and the time until a film of the tear liquid on a surface of the test contact lens being worn was broken (so-called break-up time) was measured. The average value (sec) was calculated from the measurement results for 6 lenses of the same soft contact lenses treated with the same contact lens solution. The results thereof are shown in the following Table 12. The longer average value (sec) indicates that wetting on the lens surface can be better retained.

**[Table 12]**

| | Contact Lens Used in Test | Solution No. 35 | Solution No. 13* |
|---|---|---|---|
| Average Value [sec] | Hydrous Soft Contact Lens A | 8.5 | 5.6 |
| | Hydrous Soft Contact Lens B | 8.2 | 7.6 |
| | Hydrous Soft Contact Lens C | 8.4 | 7.2 |

| | | | |
|---|---|---|---|
| * Saline | | | |

As apparent from such results shown in Table 12, it was observed that wetting on the lens surface was retained for a long period of time even when the contact lens immersed in the contact lens solution of the present invention was actually worn. Further, also for the ordinary hydrous soft contact lens including no siloxane compound as the constituent, such as hydrous soft contact lens C, it was confirmed that wettability on the surface could be effectively improved by applying the contact lens solution of the present invention.

## Claims

1. A contact lens solution comprising a copolymer of N-p-vinylbenzyl-D-lactonamide and at least one monomer copolymerizable therewith, the copolymer being present in the solution in an amount of 0.0001 to 5 w/v%.

2. The contact lens solution according to claim 1, wherein the copolymer is one selected from copolymers represented by the following formulas (1) to (4): 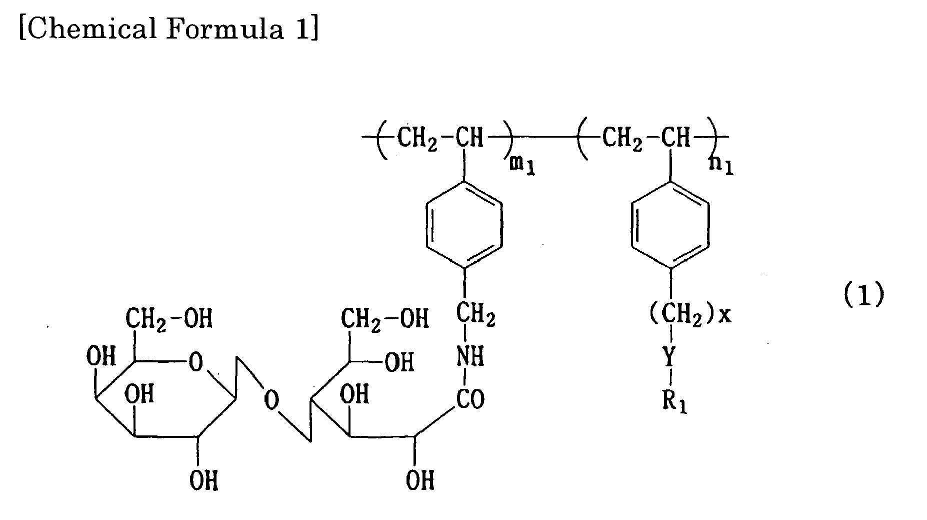 [wherein m₁:n₁=9:1 to 1:9; x represents an integer of 0 to 3; -Y- represents a single bond or an atomic group represented by -NHCO-, -NHCONH-, -CONH-, -OCONH-, -NHCOO-, -OCO-, -COO-, -CO- or -NH-; and R₁ is any one of the following (a) and (b):
(a) a saturated or unsaturated i) monocycle, ii) bicycle or iii) five-membered or six-membered heterocycle containing O atom, S atom or N atom, which has 5 to 10 carbon atoms,
wherein the monocycle, bicycle, five-membered or six-membered heterocycle may be at least partly substituted by at least one selected from the group consisting of a halogen atom, a hydroxy group, an amino group, a thiol group, a cyano group, a cyclopentyl group, a cyclohexyl group, a phenyl group and an acetyl group; and
(b) an alkyl group having 1 to 20 carbon atoms or an atomic group represented by general formula: -O-C_{α}H_{2α+1}, -S-C_{α}H_{2α+1} or -NH-C_{α}H_{2α+1} (in which α represents an integer of 1 to 20),
wherein the alkyl group or atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group]; 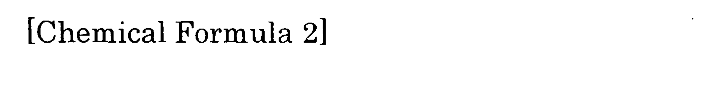 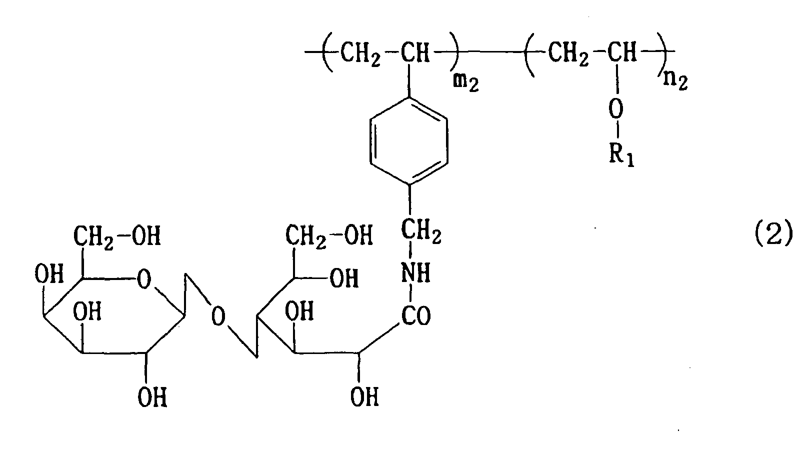 [wherein m₂:n₂=9:1 to 1:9; and R₁ has the same meaning as in formula (1)]; and, 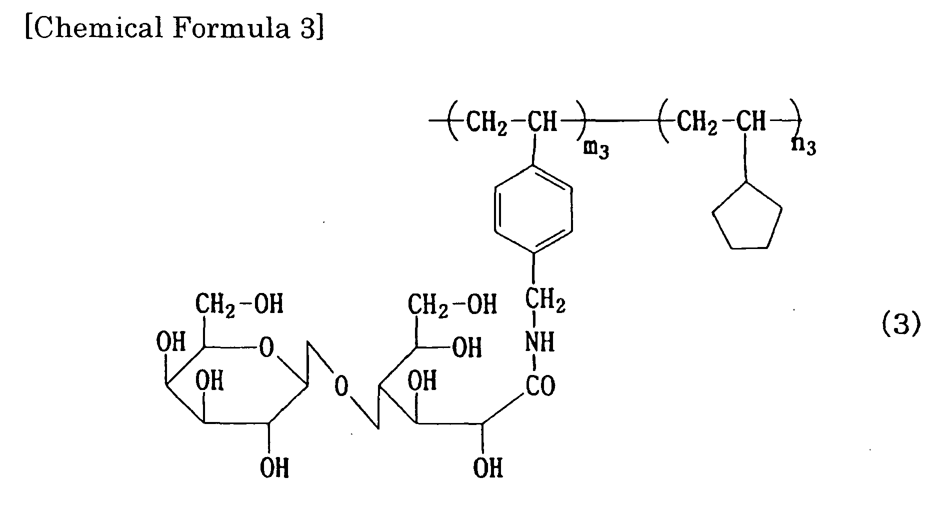 [wherein m₃:n₃=9:1 to 1:9;] 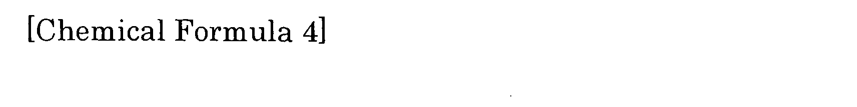 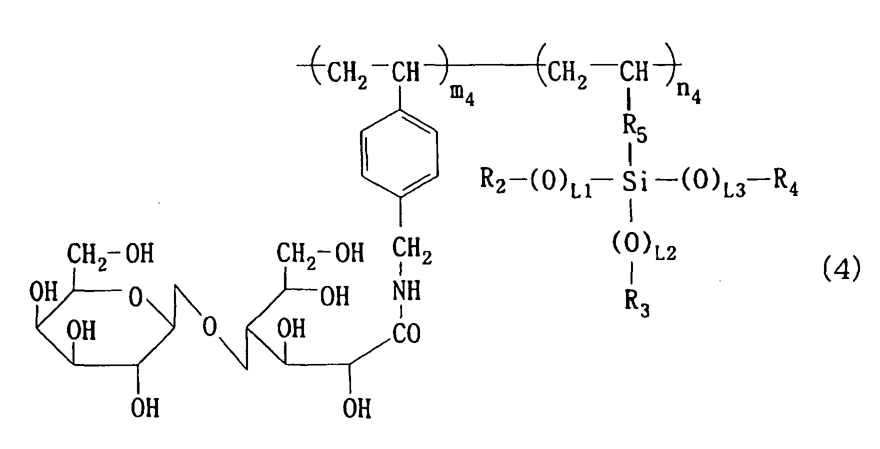 [wherein m₄:n₄=9:1 to 1:9; L1, L2 and L3 are each independently 0 or 1; and R₂, R₃ and R₄, which are independent from one another, are each an alkyl group having 1 to 20 carbon atoms or an atomic group represented by general formula: -O-C_{β}H_{2β+1}, -S-C_{β}H_{2β+1} or -NH-C_{β}H_{2β+1} (in which B represents an integer of 1 to 20),
wherein the alkyl group or atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group, and
wherein -R₅- is any one of the following (a) and (b):
(a) an atomic group represented by a single bond, or an atomic group represented by -NHCO-, -NHCONH-, -CONH-, -OCONH-, -NHCOO-, -OCO-, -COO-, -CO-, -NH- or -O-; and
(b) an atomic group represented by general formula: -C_{γ}H_{2γ}-, -O-C_{γ}H_{2γ}-, -C_{γ}H_{2γ}-O-, -S-C_{γ}H_{2γ}-, -C_{γ}H_{2γ}-S-, -NH-C_{γ}H_{2γ}- or -C_{γ}H_{2γ}-NH- (in which γ represents an integer of 1 to 20),
wherein the atomic group may be at least partly substituted by at least one selected from the group consisting of a halogen atom, a hydroxy group, an amino group, a thiol group, a cyano group, a cyclopentyl group, a cyclohexyl group, a phenyl group and an acetyl group].

3. The contact lens solution according to claim 1 or 2, further comprising a nonionic surfactant in an amount of 0.001 to 10 w/v%.

4. The contact lens solution according to any one of claims 1 to 3, further comprising a surfactant selected from the group consisting of an anionic surfactant, a cationic surfactant and an amphoteric surfactant, in an amount of 0.001 to 10 w/v%.

5. The contact lens solution according to any one of claims 1 to 4, further comprising at least one selected from the group consisting of a buffer, a tonicity agent, a thickener and a disinfectant.

6. A method for hydrophilizing a contact lens, comprising the step of bringing the contact lens solution according to any one of claims 1 to 5 into contact with the contact lens.

7. The method for hydrophilizing a contact lens according to claim 6, wherein the contact lens is immersed in the contact lens solution, and then, the contact lens is rinsed.
